# EUROPEAN PATENT SPECIFICATION

(11) **EP 2 458 989 B1**
(45) Date of publication and mention of the grant of the patent: **09.10.2013**
(21) Application number: 10737457.1
(22) Date of filing: 27.07.2010
(51) Int. Cl.: A01N 37/16, A01N 33/12, A01N 25/10, A01N 25/24, A01P 1/00, A61L 2/18

(54) **IN SITU PREPARATION OF PERACID-BASED REMOVABLE ANTIMICROBIAL COATING COMPOSITIONS AND METHODS OF USE**
IN-SITU-HERSTELLUNG VON ABLÖSBAREN ANTIMIKROBIELLEN BESCHICHTUNGSZUSAMMENSETZUNGEN AUF PERSÄUREBASIS UND ANWENDUNGSVERFAHREN
PRÉPARATION "IN SITU" DE COMPOSITIONS DE REVÊTEMENT ANTIMICROBIENNES, APTES À ÊTRE RETIRÉES, À BASE DE PERACIDE, ET PROCÉDÉS D'UTILISATION

(30) Priority: 27.07.2009 US 228774 P; 27.07.2009 US 228780 P; 27.07.2009 US 228786 P; 27.07.2009 US 228790 P; 26.07.2010 US 843091
(43) Date of publication of application: 06.06.2012
(73) Proprietor: E. I. du Pont de Nemours and Company, Wilmington, DE 19898 (US)
(72) Inventor: STAUFFER, Christina S., Wilmington Delaware 19809 (US); HOFFMANN, Christian, Newark Delaware 19711 (US); LEGER, Lynn, Mississauga Ontario L5M 5R5 (CA); ERKENBRECHER, Carl W. Jr., Elkton Maryland 21921 (US); SINICROPI, John, Rochester New York 14617 (US)
(74) Representative: Heinemann, Monica
(86) International application number: PCT/US2010/043349
(87) International publication number: WO 2011/017087

(56) References cited:
- EP-A1- 2 020 180
- WO-A1-88/00795
- WO-A1-92/19230
- WO-A1-96/03873
- WO-A1-96/19558
- WO-A1-96/22687
- WO-A1-98/20735
- WO-A2-03/028429
- US-A- 4 941 989
- ANONYMOUS: "Thickened cleaning ccmposition", RESEARCH DISCLOSURE, MASON PUBLICATIONS, HAMPSHIRE, GB, vol. 301, no. 15, 1 May 1989 (1989-05-01), XP007113670, ISSN: 0374-4353

## Description

### FIELD OF THE INVENTION

This application claims the benefit of the four United States Provisional Applications 61/228774, 61/228780, 61/228786, and 61/228790 all filed on July 27, 2009.

This invention relates to a method for controlling microorganisms comprising coating a surface with a removable, antimicrobial film-forming composition that contains peroxyacid generated *in situ* from peroxyacid-forming components and methods of applying said compositions.

### BACKGROUND

Chemical disinfectants are now used in an increasing number of industries to ensure food safety and to comply with more stringent health and safety regulations. Compositions for decontamination, disinfection and sterilization must have excellent microbiocidal efficacy, act rapidly, be non-corrosive and be effective in minimal quantities. The ideal composition must possess multiple mechanisms for destroying microorganisms, thus providing efficacy against a broad range of microorganisms and reducing the possibility of leading to evolution of disinfection-resistant microorganisms.

Compositions containing peroxide, especially hydrogen peroxide (HP) and peracetic acid (also called peroxyacetic acid, PAA) have been proven to be very effective antimicrobial agents. Many such preparations have passed the necessary tests and are registered products as sanitizers, disinfectants, and sporicides. Many of these peroxide/peroxyacid compositions are liquid solutions, which can be used for treating aqueous solutions, surfaces and objects. Some are approved for food contact surfaces and for sanitization of some food products. Some are also registered as disinfectants or sterilants as vapor phase treatments.

Methods to clean, disinfect, and/or sanitize hard surfaces, meat products, living plant tissues, and medical devices against undesirable microbial growth have been described (U.S. patent 6,545,047; U.S. patent 6,183,807; U.S. patent 6,518,307; U.S. patent application publication 20030026846; and U.S. patent 5,683,724). Peracids have also been reported to be useful in preparing bleaching compositions for laundry detergent applications (U.S. patent 3,974,082; U.S. patent 5,296,161; and U.S. patent 5,364,554).

WO 96/03873 describes a process in which an acyl donor bleach activator is reacted with a peroxygen source and the reaction product is subsequently reacted with a biocide precursor to form a biocidal compound for use as a disinfecting liquor.

Although peroxyacids have been demonstrated to be effective sporocides, bactericides and virucides, they are difficult to handle or store due to their reactive and corrosive nature and they can decompose rapidly and violently, particularly low molecular weight and high purity peroxyacids. To circumvent these drawbacks, it is therefore desirable to develop surface treatment systems where the peroxyacid is generated when it is needed to act. WO2006/016145 describes a two-pack, that is, two-container, disinfectant system, which when mixed together and diluted, provides a disinfectant solution having the active peroxyacid disinfectant present at a suitable level. The system includes a first pack (container) containing hydrogen peroxide (H₂O₂) present at a suitable pH of from 7-9.5. The second pack, having a pH of from 6-10, has an activator, which reacts with hydrogen peroxide to generate the peroxyacid.

WO2006/076334 describes a microbicidal and decontaminant composition comprising an aqueous solution of peroxides and peracids having equilibrium reaction products, a photoreactive surfactant, and a polymer, wherein said polymer interacts with said peroxides and said peracids.

US7390432 describes a two and three-part system for chemical and biological neutralization.

US5130124 describes an aqueous antimicrobial film-forming composition with H₂O₂, polyvinyl pyrrolidone, polyol, water and other components.

WO1996/022687 describes an oxidizing film-forming composition containing H₂O₂ and PAA plus polyvinyl alcohol.

As stated above, a drawback of the peroxyacid-based chemical disinfectants is their inherent lack of stability, which poses a challenge for shelf-life and when used for long term applications. Thus, a need exists for an *in situ*-generated, peracid-based, easily removable, homogenous antimicrobial coating composition providing both short-term and extended long-term antimicrobial efficacy after application to a surface.

### SUMMARY OF INVENTION

The present invention addresses the problems identified above by providing a method and composition that is antimicrobial and which also provides extended effectiveness against microorganisms by forming an antimicrobial coating on a target surface.

In one aspect the present invention is directed to a method of providing control of microorganisms at a locus comprising the steps:
a) forming a composition by combining components comprising:
   i) a water soluble or water-dispersible film-forming agent selected from polyvinyl alcohol, polyvinyl alcohol copolymer or polyvinyl pyrrolidone
   ii) an inert solvent;
   iii) a peroxyacid precursor;
   iv) a peroxygen source;
   v) a rheology modifier to provide shear-thinning properties; and
   vi) an antimicrobial agent comprising a quaternary ammonium compound
   to form an antimicrobial liquid coating composition comprising at least one peroxyacid antimicrobial agent; and
b) applying said composition obtained in step (a) to said locus; and
c) allowing said composition to dry thereby forming a coating on said locus,
wherein the shear-thinning index of the liquid coating composition is between 1.5 and 6.0.

In another aspect the present invention is directed to an antimicrobial composition as defined in claim 8.

In another aspect the present invention is directed to an article comprising a coating on at least one surface thereof of a removable antimicrobial composition as defined in claim 11.

### DETAILED DESCRIPTION

Applicants specifically incorporate the entire content of all cited references in this disclosure. Unless stated otherwise, all percentages, parts, ratios, etc., are by weight. Trademarks are shown in upper case. Further, when an amount, concentration, or other value is disclosed as either a range, preferred range or a list of preferred upper and lower values, such disclosure is to have the same effect as if each individual value within the specified range - and any range obtained from a combination of any two individual values within the disclosed range - has been specifically disclosed, even if the individual values are not uniquely or individually disclosed herein. Where a range of numerical values is recited herein, unless otherwise stated, the range is intended to include the endpoints thereof, and all integers and fractions within the range. Unless specified, it is not intended that the scope of the invention be limited to the specific values recited when defining a range.

For clarity, terms used herein are to be understood as described herein or as such term would be understood by one of ordinary skill in the art of the invention. Additional explanation of certain terms used herein, are provided below:
"Peroxygen source" refers to any peroxide compound or compound containing hydrogen peroxide that may be released in solution.
"Shear rate" refers to the velocity gradient in a flowing material and is measured in SI units of reciprocal seconds (s⁻¹).
"Shear-thinning properties" or "pseuoplastic properties" refers to a fluid that exhibits a decrease in viscosity with an increase in shear rate.
"Non-volatile" refers to a compound whose vapor pressure at 25 °C is below 1000 Pascals.
"Metal chelator" or "sequestrant" refers to agents that bind metals or metal-containing impurities and prevent their decomposition catalysis of hydrogen peroxide or peroxyacids.
"Rheology modifier" or "rheology agent" refers to compounds that increase viscosity and/or provide shear-thinning properties to a composition and cause the aqueous treatment or coating composition to cling to the surface of interest.
"Peroxyacid precursor" refers to a compound that when reacted with a peroxygen source, generates a peroxyacid.
"wt%" refers to the weight percent relative to the total weight of the solution or dispersion.
"Microorganism" is meant to include any organism comprised of the phylogenetic domains of bacteria and archaea, as well as unicellular (e.g., yeasts) and filamentous (e.g., molds) fungi, unicellular and filamentous algae, unicellular and multicellular parasites, viruses, virinos and viroids.
"Film-forming agent" or "water soluble or water dispersible coating agent", which may be used interchangeably herein, refer to agents that form a film and are employed to provide protective coating to the surface of interest. These agents are either water soluble or water dispersible. These agents are described in further detail below.
"Inert solvent or aqueous solvent" refers to water or any other solvent that facilitates application of the water dispersible coating agent and surfactant to the locus. An aqueous solvent may also be employed to rinse coated surfaces to remove the coating as needed.
"Readily removable" refers to easily removing the coatings formed after application of the liquid coating composition to the surface of interest.
"Liquid coating composition" refers to the composition comprising at least a water soluble film-forming agent, an inert solvent, a peroxyacid percursor, and a peroxygen source where at least one peroxyacid is produced.
"Antimicrobial agent" as used herein refers to a compound or substance having antimicrobial properties
"Biocide", as used herein, refers to a chemical agent, typically broad spectrum, which inactivates or destroys microorganisms. A chemical agent that exhibits the ability to inactivate or destroy microorganisms is described as having "biocidal" activity.
"Biofilm" refers to a structured community of microorganisms encapsulated within a self-developed polymeric matrix and adherent to a living or inert surface.
"Drying" refers to a process by which the inert solvent or any other liquid present in the formulation is removed by evaporation.
"Disinfectant" as used herein is a chemical that kills 99.9% of the specific test microorganisms in 10 minutes under the conditions of the test. (Germicidal and Detergent Sanitizing Action of Disinfectants, Official Methods of Analysis of the Association of Official Analytical Chemists, paragraph 960.09 and applicable sections, 15th Edition, 1990 (EPA Guideline 91-2)).
"Locus" as used herein, comprises part or all of a target surface suitable to be coated.

### Additional terms

For clarity, terms used herein are to be understood as described herein or as such term would be understood by one of ordinary skill in the art of the invention. Additional explanation of certain terms used herein, are provided below:

### Premixed components

Premixed components refer to a mixture where one or more individual components of the coating composition have been combined, but not all of the desired components of the film-forming composition are present. Additional components of the coating composition must be added to the premixed component before further use at a particular locus. In a suitable embodiment, two premixed components must be combined to form the desired coating composition according to the methods of this invention.

### Aqueous solution

Removable coatings of the present invention can be removed using an aqueous solution. An aqueous solution used for coating removal in this context is any solution containing 60 to 100 wt-% water, the remaining components being dissolved or dispersed components. Dissolved or dispersed components may include but are not limited to solvents such as alcohols, solubilizing agents, surfactants, salts, chelators, acids and bases.

### Durable

Antimicrobial coatings of the present invention are durable. Durable in this context relates to the dried coating matter remaining on the surface until its removal is purposely initiated or allowed to occur. Use conditions are the environmental conditions prevalent during the period the coating remains on the target surface for the application areas of this invention and may include inadvertent contact with water.

### Continuous

Antimicrobial coatings of the present invention can be applied as continuous or substantially continuous coatings. Continuous, or substantially continuous, in this context refers to a coating that covers the target surface without uncovered areas, coating defects, such as craters and holes or breaks.

### Multicompartment system

The components of an antimicrobial composition of the present invention can be contained in a multi-compartment system prior to combining or before use. A multicompartment system refers to the means of keeping the two or more reactive components of a multicomponent system separated before use. In one aspect, a multicompartment system comprises at least two compartments and may contain a multi-chamber dispenser bottle or a two-phase system used to combine reactive compounds in liquid form. In another aspect, powders, multi-layered tablets, or water dissolvable packets having multiple compartments, can be used for compounds in solid form or a combination of solid and liquid forms. In another aspect, any kind of system, device, container, package, bag, kit, multi-pack, dispenser, or applicator that is used to keep reactive components separated before use can be used according to the methods of this method.

### Homogeneous

Homogeneous, or substantially homogenous, in this context refers to a coating with only small thickness variations across the coating surface, with the standard deviation of the coating thickness across a coated surface being in the range of 0-40% of the coating thickness. Coatings that are not homogeneous or not substantially homogenous will not provide even antimicrobial and removal properties across the whole surface the coating is applied to and typically the appearance of inhomogeneous coatings is considered unattractive for many applications.

### Pseudoplastic index or shear thinning index (STI)

Pseudoplastic index or shear thinning index (STI) provides an indication as to how resistant to sagging and dripping the composition will be. The value recorded at the lower shear rate is divided by the value at the higher shear rate to obtain the STI. Generally, the higher the STI, the higher the resistance to sagging and dripping the coating material will have. In this disclosure the shear thinning index is defined as the ratio of the viscosity measured at a first shear rate and a second shear rate, wherein said second shear rate is 10 times the value of said first shear rate. Without being limited to specific first and second shear rates used to calculate the STI, in the Examples said first shear rate was 1 s⁻¹ and said second shear rate was 10 s⁻¹.

### Suitable reaction mixture

"Suitable reaction mixture", "components suitable for *in situ* generation of a peracid", "suitable reaction components", and "suitable aqueous reaction mixture" are used interchangeably herein and refer to materials and solution in which the peroxyacid precursor and peroxygen source come into contact. The components of the suitable aqueous reaction mixture are provided herein and those skilled in the art appreciate the range of component variations suitable for this process.

In one embodiment, the suitable reaction mixture produces peracid *in situ* upon combining the reaction components. As such, the reaction components may be provided as a multicomponent system wherein one or more of the reaction components remains separated until use. The design of systems for combining multiple active components are known in the art and generally will depend upon the physical form of the individual reaction components. For example, multiple active fluids (liquid-liquid) systems typically use multi-chamber dispenser bottles or two-phase systems (US Patent Application Pub. No. 2005/0139608; US Patent No. 5,398,846; US Patent No. 5,624,634; US Patent No. 6,391,840; E.P. Patent No. 0807156B1; US Patent Appl. Pub. No. 2005/0008526; and PCT Publication No. WO 00/11713A1) such as found in some bleaching applications wherein the desired bleaching agent is produced upon mixing the reactive fluids.

In another aspect, other forms of multi-component systems may be used to generate peracid which may include, but are not limited to those designed for one or more solid components or combinations of solid-liquid components, such as powders (e.g., many commercially available bleaching compositions, US Patent No. 5,116,575), multi-layered tablets (US Patent No. 6,210,639), water dissolvable packets having multiple compartments (US Patent No. 6,995,125) and solid agglomerates that react upon the addition of water (US Patent No. 6,319,888).

In another aspect, a suitable system for combining reactive components is use of a twin-nozzle bottle as disclosed in US Patent Application Pub. No. 2005/014427. An alternative device suitable for use with the method of the invention is a dual compartment trigger-activated fluid dispenser as disclosed in EP Patent No. 0715899B1.

In another aspect, a suitable system for mixing the suitable reaction components may be a container with a membrane separating the reactive components where upon rupturing the membrane by mechanical force, the reaction components are combined before use. In another aspect, a suitable device may be a bag-within-a-bag.

In another aspect, the means for combining or mixing the peroxyacid precursor and peroxygen source for use according to the methods of this invention include systems, devices, containers, bags, kits, multi-packs, dispensers, and applicators known to those skilled in the art that are used to keep reactive components separated before use.

In another aspect, the suitable aqueous reaction mixture may contain additional components that provide desirable functionality. These additional components include, but are not limited to buffers, detergent builders, thickening agents, emulsifiers, surfactants, wetting agents, corrosion inhibitors (e.g., benzotriazole), enzyme stabilizers, and peroxide stabilizers (e.g., metal ion chelating agents). Many of the additional components are well known in the detergent industry (see for example U.S. Pat. No. 5,932,532). Examples of emulsifiers include, but are not limited to polyvinyl alcohol or polyvinylpyrrolidone. Examples of thickening agents include, but are not limited to LAPONITE® RD, corn starch, PVP, CARBOWAX®, CARBOPOL®, CABOSIL®, polysorbate 20, PVA, and lecithin. Examples of buffering systems include, but are not limited to sodium phosphate monobasic/sodium phosphate dibasic; sulfamic acid/triethanolamine; citric acid/triethanolamine; tartaric acid/triethanolamine; succinic acid/triethanolamine; and acetic acid/triethanolamine. Examples of surfactants include, but are not limited to a) non-ionic surfactants such as block copolymers of ethylene oxide or propylene oxide, ethoxylated or propoxylated linear and branched primary and secondary alcohols, and aliphatic phosphine oxides b) cationic surfactants such as quaternary ammonium compounds, particularly quaternary ammonium compounds having a C8-C20 alkyl group bound to a nitrogen atom additionally bound to three C1-C2 alkyl groups, c) anionic surfactants such as alkane carboxylic acids (e.g., C8-C20 fatty acids), alkyl phosphonates, alkane sulfonates (e.g., sodium dodecylsulphate "SDS") or linear or branched alkyl benzene sulfonates, alkene sulfonates and d) amphoteric and zwitterionic surfactants such as aminocarboxylic acids, aminodicarboxylic acids, alkybetaines, and mixtures thereof. Additional components may include fragrances, dyes, stabilizers of hydrogen peroxide (e.g., metal chelators such as 1-hydroxyethylidene-1,1-diphosphonic acid (DEQUEST® 2010, Solutia Inc., St. Louis, Mo. and ethylenediaminetetraacetic acid (EDTA)), TURPINAL® SL, DEQUEST® 0520, DEQUEST® 0531, stabilizers of enzyme activity (e.g., polyethyleneglycol (PEG)), and detergent builders.

In another aspect, the peroxycarboxylic acid reaction product may be pre-mixed to generate the desired concentration of peroxycarboxylic acid prior to contacting the surface or inanimate object to be disinfected.

In another aspect, the peroxycarboxylic acid reaction product may be pre-mixed to generate the desired concentration of peroxycarboxylic acid and may be optionally diluted with water or a solution predominantly comprised of water, to produce a mixture with the desired lower concentration of peracid.

In another aspect, the peroxycarboxylic acid reaction product is not pre-mixed to generate the desired concentration of peroxycarboxylic acid prior to contacting the surface or inanimate object to be disinfected, but instead, the components of the reaction mixture that generate the desired concentration of percarboxylic acid are contacted with the surface or inanimate object to be disinfected, generating the desired concentration of peroxycarboxylic acid. In some embodiments, the components of the reaction mixture combine or mix at the locus. In some embodiments, the reaction components are delivered or applied to the locus and subsequently mix or combine to generate the desired peroxyacid.

The concentration of the peroxyacid precursor and the peroxygen source in the aqueous reaction mixture is chosen to obtain the desired concentration of peroxyacid in the liquid coating composition. The concentration of the peroxyacid precursor and the peroxygen source in the peroxycarboxylic acid reaction product typically ranges from 0.001 wt% to 5 wt%, more preferably between 0.01 wt% and 4 wt%, even more preferably between 0.05 wt% and 2 wt%. In one aspect, the concentration of peracid generated by the combination of the peroxyacid precursor and peroxide source is sufficient to provide an effective concentration of peracid for the desired application at a desired pH. In another aspect, the present method provides combinations of peroxyacid precursors and peroxygen sources to produce the desired effective concentration of peracid.

In on aspect, the concentration of peracid generated (e.g., peracetic acid) by the perhydrolysis of at least peroxyacid precursor is at least about 2 ppm, preferably at least 20 ppm, preferably at least 100 ppm, more preferably at least about 200 ppm peracid, more preferably at least 300 ppm, more preferably at least 500 ppm, more preferably at least 700 ppm, more preferably at least about 1000 ppm peracid, most preferably at least 2000 ppm peracid within 10 minutes, and most preferably within 5 minutes of initiating the perhydrolysis reaction.

In another aspect, the concentration of peracid generated by mixing the peroxygen precursor and peroxygen source is at least about 3000 ppm, preferably at least 5000 ppm, more preferably 8000 ppm, most preferably at least 10000 ppm within one hour, more preferably within 30 minutes of initiating the perhydrolysis reaction.

In one aspect, the reaction time required to produce the desired concentration of peracid is not greater than about two hours, preferably not greater than about 30 minutes, more preferably not greater than about 10 minutes, and most preferably not greater than about 5 minutes.

In other aspects, a hard surface or inanimate object contaminated with a concentration of a microbial population is contacted with the peracid formed in accordance with the processes described herein within about 1 minute to about 168 hours of combining said reaction components, or within about 1 minute to about 48 hours, or within about 1 minute to 8 hours, or within 1 minute to 2 hours of combining said reaction components, or any such time interval therein.

The temperature of the reaction is chosen to control the reaction rate. The temperature of the reaction may range from just above the freezing point of the reaction mixture (approximately 0 °C.) to about 75 °C., with a preferred range of reaction temperature of from about 5 °C to about 55 °C.

The pH of the final reaction mixture containing peracid is from about 2 to about 11, preferably from about 5 to about 10, more preferably from about 6 to about 9.5. The pH of the reaction mixture during the reaction or after completion of the reaction may optionally be controlled by the addition of a suitable buffer, including, but not limited to phosphate, pyrophosphate, bicarbonate, acetate, or citrate.

### Sanitizer

An antimicrobial coating composition of the present invention can be used as a sanitizer. A "sanitizer", as defined herein, is a chemical or chemical mixture that can be either (i) a food-contact sanitizer if the intention is to control microorganisms on surfaces which actually or potentially come in contact with food, or (ii) a non-food-contact sanitizer if the surfaces are not intended to come into contact with food. As defined herein, a food-contact sanitizer kills at least 99.999% of the specific test microorganisms in 30 seconds under the conditions of the test method according to EPA policy DIS/TSS-4: "Efficacy data requirements - Sanitizing rises for previously cleaned food-contact surfaces", United States Environmental Protection Agency, January 30, 1979. A non-food contact sanitizer as defined herein kills at least 99.9% of the specific test microorganisms in 5 minutes under the conditions of the method according to ASTM standard E 1153-03: "Standard Test Method for Efficacy of Sanitizers Recommended for Inanimate Non-Food Contact Surfaces", edition April 10, 2003 and published July 2003.

### Residual antimicrobial efficacy

An antimicrobial coating composition of the present invention can exhibit residual antimicrobial efficacy. "Residual antimicrobial efficacy" or "self-sanitizing properties" refers to the property of coatings formed as described herein which remain antimicrobially active after drying. The antimicrobial activity of dry coatings can be measured using the residual self-sanitizing (RSS) test method described below under General Methods. There has been a longstanding need for antimicrobial agents having improved antimicrobial efficacy and improved speed of action. The specific requirements for such agents vary according to the intended application (e.g., sanitizer, disinfectant, sterilant, aseptic packaging treatment, etc.) and the applicable public health requirements. For example, as set out in Germicidal and Detergent Sanitizing Action of Disinfectants, Official Methods of Analysis of the Association of Official Analytical Chemists, paragraph 960.09 and applicable sections, 15th Edition, 1990 (EPA Guideline 91-2), a sanitizer should provide a 99.999% reduction (5-log order reduction) within 30 seconds at room temperature (23-27 °C), against several test microorganisms.

The *in situ* enzymatically produced peracid-based, removable antimicrobial coating composition useful for the invention may be used as a replacement or a supplement for standard sanitation products (such as diluted quaternary ammonium compound solutions, peracid foams, and the like), and may be used for daily sanitation as protective coatings for equipment in use or not-in use, as well as for longer term protection (weeks or months).

The *in situ* peracid-based, removable antimicrobial coating composition of this invention provides several advantages including, but not limited to killing both free-living or planktonic microorganisms and microorganisms harbored in biofilms, reducing or preventing the growth of microorganisms by preventing the formation of biofilms and by trapping microorganisms in, beneath or otherwise in contact with the coating.

The coating composition disclosed herein are modified by formulating the composition with rheology modifiers to coat vertical, inclined, geometrically complex or hard-to-reach surfaces. This enables application of the antimicrobial agent to surfaces on or in equipment otherwise not accessible by application of conventional antimicrobial solutions with traditional shear-viscosity profiles and viscosities below about 0.01 Pascal-seconds at 25 °C. Horizontal and vertical surfaces may be covered with a thin layer of protective coating without waste of antimicrobial agent as dripping is prevented or greatly reduced by the rheology modifier. By formulating compositions with appropriate rheology modifier and degree of cross-linking, coating compositions with various coating properties may be prepared that will vary in the degree of surface finish and protection as well as ease of removal.

The coating composition of the present invention offers several mechanisms of protection against contamination of microbial or non-microbial origin, such as soiling. For example, planktonic or loosely adhering cells on the surface are killed, or alternatively growth is reduced or prevented, by the antimicrobial agent in the coating formulation as the liquid coating composition is applied.

Further, after application of the liquid antimicrobial coating composition of the present invention, cells harbored by biofilms on the surface will be killed, or growth can be reduced or prevented, by diffusion of the antimicrobial(s) into the hydrated biofilm before the applied film-forming composition completely dries to provide an antimicrobial film. For sustained antimicrobial activity it is desirable that the antimicrobial films of the present invention be semi-permeable. The antimicrobial film thus formed constitutes a reservoir of antimicrobial agent providing much longer contact time than conventional sanitary rinse solutions that typically drip off within seconds or minutes.

The long lasting activity while the coating is present on the locus is especially beneficial in a variety of applications. A film-forming antimicrobial composition of the present invention does not drip off of the target surface quickly, and is not easily removed by incidental contact, for example. The variation of film flexibility, viscosity, strength, and adhesion of the coating of the present invention permits it to be tailored to specific applications, thus making sustained antimicrobial protection available in numerous situations where such sustained activity (residual benefit) was not previously available.

### COMPONENTS OF THE COMPOSITION

The following provides a detailed description of the components of the film or coating described herein.

### Film-forming water soluble or water dispersible agent

The film-forming water soluble or water dispersible agent may be at least one of any agent, as described below, that is durable and removable. A film of the present invention is designed to be removable under relatively mild conditions. For example, a film of the present invention can be removed when subjected to treatment with an aqueous solution at a temperature above 15 °C, preferably above 30 °C. The film-forming agents are selected from, polyvinyl alcohols, polyvinyl alcohol copolymers and polyvinyl pyrrolidones. The composition may further comprises film-forming agents such as polyacrylic acid, acrylate homopolymers and copolymers, ionic hydrocarbon polymers, polyurethanes, polysaccharides, functionalized polysaccharides, arabinoxylanes, glucomannanes, guar gum, gum arabic, johannistree gums, cellulose, methyl cellulose, ethyl cellulose, hydroxymethyl cellulose, hydroxyethyl cellulose, carboxymethyl cellulose, carboxyethyl cellulose starch, hydroxyethyl starch, xanthan gum, carrageenan, curdlan, pullulan, gelatin, dextran, chitosan, glycerol, sodium alginate, sodium alginate cross-linked with calcium salt, carrageenan, ethyleneoxide/propylene oxide/ethyleneoxide block copolymers, and combinations there of. One skilled in the art may easily select the range of suitable molecular weights in order to provide a range of water solubility to provide a readily removable coating according to the methods of this invention.

### Polyvinyl alcohol and copolymers thereof

Polyvinyl alcohol, sometimes referred to as poly(vinyl alcohol), is made from polyvinyl acetate by hydrolysis. The physical properties of polyvinyl alcohol are controlled by the molecular weight and the degree of hydrolysis. The most commonly available grades of polyvinyl alcohol, ranked by degree of hydrolysis, are an 87-89% grade (containing 11-13 mol% residual vinylacetate units), a 96% hydrolysis grade (containing 4 mol% residual vinyl acetate units), and the "fully hydrolyzed" and "superhydrolyzed" grades, which are about 98% and greater-than-99% hydrolyzed, respectively. Lower degrees of hydrolysis (e.g., 74% and 79%) are also commercially available. Some preferred degrees of hydrolysis are greater than 85 mol%, or greater than 92 mol%. The polyvinyl alcohol component of the present invention may also be a copolymer of vinyl alcohol, such as one obtained by hydrolyzing a copolymer of vinyl acetate with small amounts (up to about 15 mol%) of other monomers. Suitable co-monomers are e.g., esters of acrylic acid, methacrylic acid, maleic or fumaric acids, itaconic acid, etc. Also, copolymerization of vinyl acetate with hydrocarbons e.g., alpha-olefins such as ethylene, propylene or octadecene, etc., with higher vinyl esters such as vinyl butyrate, 2-ethyl hexoate, stearate, trimethyl acetate, or homologues thereof ("VV-10" type of vinyl esters sold by Shell Chem. Co.), etc. gives copolymers that may be hydrolyzed to suitable polyvinyl alcohol copolymers. Other suitable co-monomers are N-substituted acrylamides, vinyl fluoride, allyl acetate, allyl alcohol, etc. Also the free unsaturated acids such as acrylic acid, methacrylic acid, monomethyl maleate, etc. may act as co-monomers.

Because of the variety of grades either known in the literature or commercially available, one skilled in the art may formulate a polyvinyl alcohol solution having an average degree of hydrolysis ranging from 74 to more than 99% simply by blending the known or commercial grades in any desired ratios. Accordingly, the term "partially hydrolyzed grade polyvinyl alcohol", as used in this description should be understood to include both a single grade and a mixture of grades, and the term "average degree of hydrolysis" should be understood to refer to the degree of hydrolysis arrived at by averaging (with appropriate weighting on the basis of proportions) the partially hydrolyzed grades in the mixture, if a mixture is used, or the average degree of hydrolysis of a single grade, if a single grade is used (an "88% grade", for example, may be the average of a spectrum ranging from 87 to 89% within the same grade).

Film flexibility, water sensitivity, ease of solvation, viscosity, film strength and adhesion of the polyvinyl alcohol film may be varied by adjusting molecular weight and degree of hydrolysis.

In one embodiment, the polyvinyl alcohol for use in the process of this invention has a degree of hydrolysis from about 85% to greater than 99%. In another embodiment, the polyvinyl alcohol has a degree of hydrolysis from about 87% to greater than 89%. In one embodiment, the polyvinyl alcohol has a number-averaged molecular weight (Mn) in g/mol in the range of between about 4,000 to about 200,000, or about 4,000 to about 150,000, or 10,000 to about 100,000.

In one embodiment, the polyvinyl alcohol has a molecular weight that falls in the range of between about 10,000 and 130,000. In another embodiment, the polyvinyl alcohol of various molecular weights may be blended to give the desired properties.

In one embodiment, the polyvinyl alcohol is used at about 2% to about 30% by weight of the weight of the solution. In a more specific embodiment, the polyvinyl alcohol is used at about 2% to about 15% by weight of the weight of the solution. In an even more specific embodiment, the polyvinyl alcohol is used at about 5% to about 12% by weight of the weight of the solution.

### Polyvinylpyrrolidone (PVP)

The film-forming composition of the present invention may contain PVP at a concentration of about 0.25 to about 50% by weight. Suitable grades of PVP are available from International Specialty Products (Wayne, NJ, USA). Such grades include: K-15, having a weight-average molecular weight (Mw) in g/mol in the range of about 6,000 to about 15,000; K-30, having a molecular weight range of about 40,000 to about 80,000; K-60, having a molecular weight range of about 240,000 to about 450,000; K-90, having a molecular weight range of about 900,000 to about 1,500,000; and K-120, having a molecular weight range of about 2,000,000 to about 3,000,000. Mixtures of PVP's may be employed, as may combinations of PVP and other film-forming compounds.

Typically, lower molecular weight PVP will give a less viscous product than a higher molecular weight PVP at the same concentration. For a given concentration of PVP, as the molecular weight range increases, the viscosity will also increase. The present invention may employ PVP having any of a number of molecular weight ranges. For example, film-forming compositions may utilize the PVP grades K-15, K-30, K-60, K-90, or K-120 described above. It is preferred, however, to use PVP with a molecular weight distribution between about 15,000 and about 3,000,000 g/mol. PVP having this molecular weight distribution typically gives a film-forming composition with a viscosity, which may be easily adjusted and washes off a surface easily with no visible signs of interaction with a painted surface. In a preferred embodiment, PVP with a molecular weight distribution between about 15,000 and about 3,000,000 g/mol is present at a concentration of between about 0.25% and about 40% by weight. In another preferred embodiment, PVP with a molecular weight distribution between about 30,000 and about 1,200,000 g/mol is present at a concentration of between about 0.25% and about 10% by weight.

### Peroxaycids

Peroxyacids are widely recognized as highly effective disinfectants. Peroxyacetic acid (also referred to as peracetic acid or PAA) is particularly suitable for use in the practice of the present invention. There are disadvantages with using peroxyacids, conventionally supplied in an equilibrium mixture, owing to their inherent instability. Some of the disadvantages can be overcome by *in situ* generation of the peroxyacid. *In situ* generation has advantages in that the amount of peroxyacid produced may be stoichiometrically controlled through selecting the relative composition of the starting materials. Moreover, higher concentrations of peroxyacid can be obtained than are available from equilibrium systems due to the non-equilibrium nature of the *in situ* systems.

To provide *in situ* generation of a peroxyacid, a system in which the reactants, that is the peroxyacid precursor and the peroxygen source, are maintained in separate compartments or containers until required such as described in WO 2006/016145 is suitable for use in the practice of the present invention.

In the present invention a film-forming component is combined with other components, including a peroxyacid precursor and a peroxygen source. The film-forming component and the peroxyacid precursor can be stored together in one container or pack, or stored in separate packs, prior to combining to form the antimicrobial coating composition and application to a target surface. For example, the film forming component can be stored in a separate pack or container from the peroxyacid precursor and the peroxygen source, and combined just prior to application to the target surface or, alternatively, the film-forming component can be stored together with the peroxyacid precursor but separately from the peroxygen source until such time as they are combined and applied to a target surface.

### Peroxyacid precursors

Typically, in order to generate the peroxyacid, a peroxyacid precursor is reacted with a peroxygen source, usually hydrogen peroxide. Examples of peroxyacid precursors -often referred to as activators or bleaching activators- are molecules containing acyl group donors such as N-acyl amides (including lactams), acyl halides, or O-acyl esters, particularly gem-diesters, but also including lactones, may be used.

In the present disclosure, the compound of the formula I may be any N-acyl or O-acyl donor compound, in which L is a leaving group attached via an oxygen or nitrogen atom to the carbonyl carbon atom and R is an alkyl or aryl group. The compound of the formula I may be an ester or, even more preferably, an amide derivative. Amide derivatives include N-acyl lactams, such as N-acetyl-caprolactam and N,N-diacyl amides, such as tetraacetylethylenediamine (TAED). 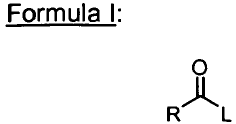 Other examples of N-acyl derivatives suitable for use herein are:
a) 1,5-diacetyl-2,4-dioxohexahydro-1,3,5-triazine (DADHT);
b) Acylated glycolurils, such as tetraacetyl glycoluril and tetraproprionyl glycoluril;
c) Diacylated 2,5-diketopiperazines, such as 1,4-diacetyl-2,5 diketo-piperazine;
d) N,N'-diacetyl-N,N'-dimethylurea (DDU);
e) Alpha-acyloxy-(N,N')polyacyl malonamides, such as alpha-acetoxy-(N,N')-diacetyl malonamide;
f) N-acyl lactams, such as N-benzoylcaprolactam, N-acetyl-caprolactam, the analogous compounds formed from C4-10 lactams.
g) N-acyl derivatives of substituted or unsubstituted succinimide, phthalimide and of imides of other dibasic carboxylic acids, having 5 or more carbons.
h) Tetraacetylethylenediamine (TAED)
Alternatively the compound may be an ester, for instance:
i) Sugar esters, such as pentaacetylglucose (PAG);
j) Esters of imidic acids such as ethyl benzimidate;
k) triacylcyanurates, such as triacetylcyanurate and tribenzoylcyanurate,
I) Esters giving relatively surface active oxidizing products, for instance, alkanoyloxybenzenesulfonates, compounds of the formula I where L comprises an aryl group having a sulphonic acid group (optionally salified) substituted in the ring to confer water solubility on a benzyl group, especially nonanoyloxybenzenesulphonate sodium salt (NOBS), isononanoyloxybenzenesulphonate sodium salt (ISONOBS) and benzoyloxybenzenesulphonate sodium salt (BOBS).
m) Geminal diesters of lower alkanoic acids and gem-diols, such as those described in EP-A-0125781 especially 1,1,5-triacetoxypent-4-ene and 1,1,5,5-tetraacetoxypentane and the corresponding butene and butane compounds, ethylidene benzoate acetate and bis(ethylidene acetate) adipate;
n) Glycerides, such as triacetin and glyceryl trioctanoate. The activator is usually provided as a source of peroxyacid precursor.

Any of the above peroxyacid activators may be used, either alone or in combination with other activators, in the present disclosure.

The preferred peroxyacid activators in this context are the N-acyl amides. The more preferred N-acyl activators are tetraacetylethylene-diamine (TAED) and N-acetylcaprolactam.

### Peroxygen source

In order to generate a peroxyacid, the most commonly used source of the peroxide moiety is hydrogen peroxide itself. The peroxyacid is generated through the reaction of hydrogen peroxide via a nucleophilic pathway, with an acyl group containing molecule.

The peroxygen source may be any peroxide compound and may be selected from among the following: hydrogen peroxide, sodium perborate monohydrate, sodium perborate tetrahydrate, sodium pyrophosphate peroxyhydrate, urea peroxyhydrate, sodium percarbonate, sodium peroxide and mixtures thereof. Other compounds, related to those mentioned above, that release hydrogen peroxide upon contact with an aqueous solution, may also be used if they are stable enough for the intended use and desired shelf life.

The preferred peroxygen source is an inorganic persalt, sodium percarbonate.

### Solvents

Inert solvents useful for the invention include: water; alcohols, preferably containing from about 1 to about 6 carbon atoms and from 1 to about 6 hydroxyl groups; glycols; polyglycols; glycol ethers; and polyoxides. Examples include ethanol, isopropanol, n-propanol, 1,2-propanediol, 1,2-butanediol, 2-methyl-2,4-pentanediol, mannitol and glucose, and propylene glycol ethers. Also useful are the free acids and alkali metal salts of sulfonated alkylaryls such as toluene, xylene, cumene and phenol or phenol ether or diphenyl ether sulfonates; alkyl and dialkyl naphthalene sulfonates and alkoxylated derivatives.

### Additional performance-enhancing agents

### Surfactants

The compositions useful for the present invention may also contain one or more surfactants. While not being bound by theory, it is believed that a surfactant will aid wetting of the surface to be covered and will aid even coverage by the film. The surfactant is also believed to aid foaming by the film when removed, thereby aiding removal of the film and washing of the protected surface. Suitable surfactants have a preferred hydrophilic-lipophilic balance (HLB) of from about 9 to about 17. Suitable surfactants include, but are not limited to: amphoteric surfactants, such as Amphoteric N from Tomah Products; silicone surfactants, such as BYK 348 available from BYK Chemie (BYK-Chemie GmbH, Wesel, Germany); fluorinated surfactants such as Zonyl® FS300 from DuPont (DuPont, Wilmington, DE, USA); and nonylphenoxypolyethoxyethanol based surfactants, such as Triton N-101 available from Dow (Midland, MI, USA). Other suitable surfactants include ethoxylated decynediols such as Surfynol 465 available from Air Products & Chemicals (Allentown, PA, USA); alkylaryl polyethers such as Triton CF-10 available from Dow; octylphenoxy polyethoxy ethanols such as Triton X-100 available from Dow; ethoxylated alcohols such as Neodol 23-5 or Neodol 91-8 available from Shell (The Hague, the Netherlands); Tergitol 15-S-7 available from Dow; Steol-4N, a 28% sodium laureth sulfate from Stepan Company (Northfield, IL, USA); amine oxides such as Ammonyx® LO available from Stepan; EO/PO block copolymers such as Pluronic® 17R4 available from BASF (Parsippany, NJ, USA); sorbitan derivatives such as Tween 20 or Tween 60 from Uniqema (New Castle, DE, USA); and quaternary ammonium compounds, such as benzalkonium chloride.

Other suitable surfactants include organo-silicone surfactants such as Silwet®L-77 from Setre Chemical Company (Memphis, TN, USA); DowCorning® Q2-5211 from DowCorning Silicones (Midland, MI, USA); or Silsurf® A008 by Siltech Corporation (Toronto, ON, Canada).

### Plasticizers

It is important for flexibility and integrity of the protective film that the resultant film be plasticized. Plasticization of the film has been accomplished for the purposes of this invention by the incorporation of a suitable plasticizing agent such as polyethylene glycol or glycerol. Other plasticizers suitable for the invention include, but are not limited, to solvents, polyols, polyethylene glycols of and average molecular weight between 200 and 800 g/mole and sorbitol. PEG is preferred over glycerol since glycerol is easily metabolized by microorganisms potentially resulting in microbial growth.

Inclusion of a plasticizer generally also allows the film to retain a slightly tacky surface feel. As the plasticizer level increases, the resulting film will also exhibit an increasing degree of tackiness. Such tackiness may be desirable at low levels in order to capture airborne particles and soil or other materials. If plasticizer levels are too high, however, the coating becomes too tacky and will show low resistance to accidental mechanical removal, by wiping, for example.

The preferred plasticizer amount is from about 1 wt% to about 20 wt% of the weight of the film former, and more preferably from about 5 wt% to about 10 wt%.

### Rheology modifiers

The composition useful for the invention contains one or more rheology modifiers employed to provide shear-thinning properties. Rheology modifiers can also enhance viscosity, or thicken and cause the aqueous treatment or coating composition to cling to the surface. Clinging enables the composition to remain in contact with transient and resident microorganisms for longer periods of time, promoting microbiological efficacy and resisting waste because of excessive dripping. The rheology modifier may be a film former or act cooperatively with a film-forming agent to form a barrier that provides additional protection. Water soluble or water dispersible rheology modifiers that are useful may be classified as inorganic or organic. The organic thickeners may further be divided into natural and synthetic polymers with the latter still further subdivided into synthetic natural-based and synthetic petroleum-based.

Inorganic thickeners are generally compounds such as colloidal magnesium aluminum silicate (VEEGUM®), colloidal clays (Bentonites), or silicas (CAB-O-SIL®) which have been fumed or precipitated to create particles with large surface to size ratios. Natural hydrogel thickeners of use are primarily vegetable derived exudates. For example, tragacanth, karaya, and acacia gums; and extractives such as carrageenan, locust bean gum, guar gum and pectin; or, pure culture fermentation products such as xanthan gum are all potentially useful in this invention. Chemically, all of these materials are salts of complex anionic polysaccharides. Synthetic natural-based thickeners having application are cellulosic derivatives wherein the free hydroxyl groups on the linear anhydro-glucose polymers have been etherified or esterified to give a family of substances which dissolve in water and give viscous solutions. This group of materials includes the alkyl and hydroxyl- alkylcelluloses, specifically methylcellulose, hydroxyethylmethylcellulose, hydroxypropylmethylcellulose, hydroxybutylmethylcellulose, hydroxyethylcellulose, ethylhydroxyethylcellulose, hydroxypropylcellulose, and carboxy- methylcellulose. Another preferred group of thickeners include polyacrylates such as the proprietary Acusol thickeners, (e.g., Acusol 823, Rohm and Haas, Philadelphia, PA, USA), and Carbopol thickeners, such as Carbopol 934 or Carbopol Aqua-30 Polymer (B F Goodrich, Cleveland, OH, USA). Additional preferred acrylate-based rheology modifiers include the proprietary cationic Rheovis® thickeners (Ciba, Basel, Switzerland). A polyacrylate thickener may be used at concentrations of up to about 3 wt% of the film former weight. Mixtures of thickening agents may also be employed where the total amount may be up to about 3 wt% depending on the thickeners used and the desired viscosity of the final product.

Other potential thickeners for this application include dextrin, cornstarch and hydrous magnesium silicates, such as sodium magnesium silicate sold under the trade name Laponite XLG (Southern Clay Products, Inc., Gonzales, TX, USA).

### Antimicrobial agents

In addition to the *in situ* generated peroxyacid and the quaternary ammonium compound, one or more additional antimicrobial agents may be present in the composition. The antimicrobial agent useful for the invention may be either an inorganic or organic agent, or a mixture thereof.

The term "inorganic antimicrobial agent" used herein is a general term for inorganic compounds which contain a metal or metal ions, such as silver, zinc, copper and the like which have antimicrobial properties.

The invention is not to be limited to the selection of any particular antimicrobial agent, and any known water-soluble or water-dispersible antimicrobial may be included in the compositions of the invention such as antimicrobials, mildewcides, antiseptics, disinfectants, sanitizers, germicides, algicides, antifouling agents, preservatives, and combinations of the foregoing and the like provided that the antimicrobial agent is chemically compatible with other components in the composition. Suitable classes of antimicrobial agents are described below.

Examples of useful antimicrobial agents include chlorhexidine, chlorhexidine gluconate, glutaral, halazone, hexachlorophene, nitrofurazone, nitromersol, thimerosol, C1-C5 -parabens, hypochlorite salts, clofucarban, clorophen, phenolics, mafenide acetate, aminacrine hydrochloride, quaternary ammonium salts, chlorine and bromine release compounds (e.g., alkali and alkaline earth hypochlorites and hypobromites, isocyanurates, chlorinated derivatives of hydantoin, sulfamide, amine, etc.), peroxide and peroxyacid compounds (e.g., peracetic acid, peroctanoic acid), protonated short chain carboxylic acids, oxychlorosene, metabromsalan, merbromin, dibromsalan, glyceryl laurate, sodium and/or zinc pyrithione, trisodium phosphates, (dodecyl)(diethylenediamine)glycine and/or (dodecyl) (aminopropyl)glycine and the like. Useful quaternary ammonium salts include the N-C₁₀-C₂₄ - alkyl-N-benzyl-quaternary ammonium salts which comprise water solubilizing anions such as halide, e.g., chloride, bromide and iodide; sulfate, methosulfate and the like and the heterocyclic imides such as the imidazolinium salts. Quaternary ammonium salts may also include other non-halogenated anions such as propionates and saccharinates and the like. Useful phenolic germicides include phenol, m-cresol, o-cresol, p-cresol, o-phenyl-phenol, 4-chloro-m-cresol, chloroxylenol, 6-n-amyl-m-cresol, resorcinol, resorcinol monoacetate, p-tert-butylphenol and o-benzyl-p-chlorophenol. Useful antimicrobial agents known to be effective in preventing the visible growth of mildew colonies, include, for example, 3-iodo-2-propynl butylcarbamate, 2-(4-thiazolyl)benzimidazole, diiodomethyl-p-tolylsulfone, tetrachloroisophthalonitrile, the zinc complex of 2-pyridinethiol-1-oxide (including salts thereof) as well as combinations of the foregoing. The coating composition comprising the antimicrobial agent offers protection against diverse microorganisms.

In one embodiment, the coating composition protects against Gram positive or Gram negative bacteria. Gram positive bacteria which are inhibited or killed by the coating include, but are not limited to, *Clostridium tetani, C. perfringens, C. botulinum,* other *Clostridium* species, *Mycobacterium tuberculosis, M. bovis, M. typhimurium, M. bovis strain BCG, BCG substrains, M. avium, M. intracellulare, M. africanum, M. kansasii, M. marinum, M. ulcerans, M. avium subspecies paratuberculosis, Staphylococcus aureus, S. epidermidis, S. equi, Streptococcus pyogenes, S. agalactiae, Listeria monocytogenes, L. ivanovii, Bacillus anthracis, B. subtilis, Nocardia asteroides, and other Nocardia species, Streptococcus viridans group, Peptococcus species, Peptostreptococcus species, Actinomyces israelii* and other *Actinomyces* species, *Propionibacterium acnes,* and *Enterococcus* species. Gram negative bacteria which are inhibited or killed by the coating include, but are not limited to, *Pseudomonas aeruginosa,* other *Pseudomonas* species, *Campylobacter* species, *Vibrio cholerae, Ehrlichia species*, *Actinobacillus pleuropneumoniae, Pasteurella haemolytica, P. multocida,* other *Pasteurella* species, *Legionella pneumophila,* other *Legionella* species, *Salmonella typhi,* other *Salmonella* species, *Shigella* species *Brucella abortus,* other *Brucella* species, *Chlamydia trachomatis, C*. *psittaci, Coxiella burnetti, Neiserria meningitidis, N. gonorrhea, Haemophilus influenzae, H. ducreyi,* other *Haemophilus* species, *Yersinia pestis, Y*. *enterolitica,* other *Yersinia* species, *Escherichia coli, E. hirae* and other *Escherichia* species, as well as other Enterobacteriacae, *Burkholderia cepacia, B. pseudomallei, Francisella tularensis, Bacteroides fragilis, Fusobacterium nucleatum, Provetella* species, *Cowdria ruminantium, Klebsiella* species, and *Proteus* species.

In another embodiment, the coating provides protection against fungi, including but are not limited to, *Alternaria alternata, Aspergillus niger, Aureobasidium pullulans, Cladosporium cladosporioides, Drechslera australiensis, Gliomastix cerealis, Monilia grisea, Penicillium commune, Phoma fimeti, Pithomyces chartarum,* and *Scolecobasidium humicola.* Chelating agents

Trace amounts of impurities, especially metals, may react with hydrogen peroxide and peroxyacids and cause decomposition. Therefore, many peroxide/peroxyacid compositions include stabilizing ingredients, such as compounds that sequester metals and metal-containing impurity materials. Examples of preferred chelating agents include, but are not limited to, alkylideneaminophosphonic acids or salts thereof, some of which are marked under the name Dequest® (Thermphos, Switzerland), 1-hydroxyethylidene-1,1-diphosphonic acids and salts thereof, some of which are marked under the name Turpinal® (Thermphos), and 2-phosphono-1,2,4-butanetricarboxylic acids and salts thereof, available from LANXESS Corporation (Pittsburgh, PA, USA) under Bayhibit®. A further class of compounds suitable for use are the aminocarboxylicacids or salts thereof. An example is ethylenediaminetetraacetic acid (EDTA).

In addition, other suitable chelating agents include dipicolinic acid, ethane-1,1,2-triphosphonic acid and ethylidene-1,1-diphosphonic acid. Furthermore, also well know in the art as chelating agents are phosphates, polyphosphates, pyrophosphates, and carboxylic acids, such as citric acid or salicylic acid. The stabilizer or stabilizers should be present in sufficient amounts to inhibit breakdown of the peroxide/peroxyacids.

### Colorants or Dyes

Colorants useful for the present invention include dyes and pigments such as food grade pigments.

Dyes useful for the invention include both water soluble and water insoluble dyes. Water soluble dyes may be formulated easily in the aqueous systems of the invention. Water insoluble dyes may be included in an oil phase that may be dispersed or suspended in the antimicrobial coating compositions useful for the invention. Useful dyes for the purpose of this invention are typically organic compounds that absorb visible light resulting in the appearance of a detectable color. Fluorescent dyes may also be used, for example, for purposes of visualizing a film by ultraviolet light.

The dyes typically useful in this invention are colorants approved for use in foods, drugs, cosmetics and medical devices. Colorants currently in use and their status is as follows: Colorants permitted in foods that are (1) subject to certification: FD&C Blue No. 1, FD&C Blue No. 2, FD&C Green No. 3, FD&C Red No. 3, FD&C Red No. 40, FD&C Yellow No. 5, FD&C Yellow No. 6, Citrus Red No. 2, and Orange (B) (2) exempt from certification: annatto extract, theta-apo-8'-carotenal, canthaxanthin, caramel, theta-carotene, carrot oil, cochineal extract (carmine), corn endosperm oil, dehydrated beets (beet powder), dried algae meal, ferrous gluconate, fruit juice, grape color extract, grape skin extract, paprika, paprika oleoresin, riboflavin, saffron, synthetic iron oxide, tagetes meal and extract, titanium dioxide, toasted partially defatted cooked cottonseed flour, turmeric, termeric oleoresin, ultramarine blue, and vegetable juice. Colorants permitted in drugs (including colorants permitted in foods) that are (1) subject to certification: FD&C Red No. 4, D&C Blue No. 4, D&C Blue No. 9, D&C Green No. 5, D&C Green No. 6, D&C Green No. 8, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Red No. 39, D&C Violet No. 2, D&C Yellow No. 7, D&C Yellow No. 8, D&C Yellow No. 10, D&C Yellow No. 11, and Ext. D&C Yellow No. 7. Additionally cantaxanthin, beta carotene, chlorophyllin, and other colors are known. For a more detailed listing and/or discussion on approved colors, see D. M. Marmion, Handbook of U.S. Colorants, Foods, Drugs, Cosmetics and Medical Devices, John Wiley & Sons Inc., New York (1991) and U.S. Code of Federal Regulations, Title 21, parts 70-82.

### Cross-linking agents

The present invention may optionally include cross-linking agents. Advantages of using cross-linking agents with the film-forming composition include influencing the mechanical film properties, such as tackiness and mechanical strength, as well as solubility of the coating. Furthermore, cross-linking decreases tackiness and prevents soil and microorganisms from physically adhering to the polymer film, which may be desirable for certain applications. The degree of cross-linking is adjusted so to achieve the desired combination of properties.

Cross-linking agents suitable for use with polyvinyl alcohol and copolymers thereof include, but are not limited to: aldehydes (e.g., formaldehyde, glyoxal, glutaraldehyde), boric acid, sodium tetraborate, metal ions (e.g., ions of Zn, Fe, Al, Ni, V, Co, Cu, Zr, Ti, Mn), organometallic compounds (e.g., organic titanates such as DuPont Tyzor®, organic Cr(III) complexes such as DuPont Quilon®), siloxanes (e.g., tetraethoxysilane, polydimethylsiloxane), isocyanates (e.g., of the blocked, water-soluble or dispersed type), epoxides (e.g., diglycidyl ether), dicarboxylic acid (e.g., oxalic, maleic, fumaric, phthalic), urea based crosslinkers (e.g., Sunrez 700). Bi- and trivalent metal cations (e.g., Fe (II), Fe (III), Al (III)) are preferred because they provide the formation of a coordinative linkage between the PVOH polymer chains upon film drying. This allows the cross-linker to be added to the film-forming liquid in a 'one-pot' mixture. Care must be taken to choose an adequate concentration in order to efficiently cross-link the polymer without precipitating other ingredients such as particulate rheology control agents.

In most cases the cross-linking agent will be mixed with other ingredients using standard mixing techniques. The cross-linking reaction may optionally be carried out in the presence of a catalyst, as is well known to those skilled in the art. In the case of the aldehydes, isocyanates, siloxanes, diglycidyl ether, and dicarboxylic acid, heat and an acid catalyst or metal catalyst may be used additionally.

The cross-linking agent concentration in the formulation may be zero to an upper limit which is either determined by the stability limit of the formulation where precipitation starts to occur, or the inability of the resulting film to be removed efficiently.

The preferred cross-linking agent concentration may depend strongly on the type of cross-linking agent used and is typically below 25 wt% of the polymer content, more preferably below 10 wt% of the polymer content.

In addition to the foregoing components, the composition of the present invention may also comprise one or more performance enhancing additives also known as "performance enhancers". These include flash rust inhibitors, which include any of a number of organic or inorganic materials used in a water-based system to prevent rust from forming on contact with the material and bare metal. Two examples are sodium benzoate or benzotriazole.

Another optional performance enhancing additive includes one or more of an array of defoamers recommended for water-based systems, to prevent unwanted foaming of the product during application. Too much foam may disrupt the required continuous film formation of the product and result in product failure. It may also be advantageous to add a foam control product, to aid in mixing and processing the masking composition, such as Drewplus L475 from Ashland Chemical, Inc. Drew Industrial Division (Covington, KY, USA). Additional optional performance enhancing additives are antioxidants to increase the shelf life of the coating formulation. One example is butylated hydroxytoluene. Further additional additives include fragrances.

Foaming agents may additionally be added to create gas bubbles in the applied coating. Gas bubbles may function as an opacifying agent to facilitate the application and/or to allow for longer contact time with a surface; e.g., by preventing dripping from an inclined surface and/or to reduce the amount of coating formulation needed to treat a certain surface area or volume.

Application indicators may also be added. Some of these are described above, but include pigments, dyes, fluorescent dyes, pH indicators or gas bubbles generated during application.

Small amounts (typically less than 1 percent by weight) of these additional materials may be added with an appropriate adjustment of the water or other components. It is to be understood that mixtures of any one or more of the foregoing optional components may also be employed.

For loci comprised of fibrous substrates, an optional performance-enhancing ingredient is an agent that provides a surface effect. Such surface effects include no iron, easy to iron, shrinkage control, wrinkle free, permanent press, moisture control, softness, strength, anti-slip, antistatic, anti-snag, anti-pill, stain repellency, stain release, soil repellency, soil release, water repellency, oil repellency, odor control, antimicrobial, or sun protection.

### Applying the antimicrobial coating composition

The film or coating may be applied to the target surface or locus by any means, including pouring. The film or coating is applied to achieve a continuous and/or homogenous layer on a target surface. Coating systems routinely used for paints and coatings, such as, but not limited to, brushes, rollers, paint pads, mats, sponges, combs, hand-operated pump dispensers, compressed air operated spray guns, airless spray guns, electric or electrostatic atomizers, backpack spray application equipment, aerosol spray cans, clothes, papers, feathers, styluses, knives, and other applicator tools may be used for coating. If dipping is used as a method to apply the coating, no special equipment is required. If an aerosol spray can is used for application, the coating composition can be mixed with an aerosol propellant (such as a compressed gas) or the coating composition can be physically separated from the propellant by a barrier material such as a polymer bag inside the can; if the coating composition and the propellant are mixed the mixture can constitute one or more liquid phases.

For fibrous substrates, such as textiles and carpets, the coating may be applied by exhaustion, foam, flex-nip, nip, pad, kiss-roll, beck, skein, winch, liquid injection, overflow flood, roll, brush, roller, spray, dipping, immersion, and the like. The coating may also be applied by use of the conventional beck dyeing procedure, continuous dyeing procedure or thread-line application.

In one embodiment of the current disclosure, electrostatic sprayers can be used to coat the surface. Electrostatic sprayers impart energy to the aqueous coating composition via a high electrical potential. This energy serves to atomize and charge the aqueous coating composition, creating a spray of fine, charged particles. Electrostatic sprayers are readily available from suppliers such as Tae In Tech Co., South Korea and Spectrum, Houston, TX, USA.

In another embodiment of the invention, an airless spray gun may be used to apply the coating to the target surface. Airless spray guns use high fluid pressures and special nozzles, rather than compressed air, to convey and atomize the liquid. The liquid is supplied to an airless gun by a fluid pump at pressures typically ranging from 3.5 to 45 MPa. When the paint exits the fluid nozzle at this pressure, it expands slightly and atomizes into tiny droplets without the impingement of atomizing air. The high velocity of the exiting paint propels the droplets toward the target surface. The fluid nozzle on an airless gun differs substantially from the fluid nozzle on an air atomized gun. Selection of the proper nozzle determines how much paint is delivered and the fan pattern of application. The size of the airless nozzle orifice determines the quantity of paint to be sprayed. Airless fluid delivery is high, ranging from 700-2000 mL/min. Recommended gun distance is about 30 cm from the target, and depending upon the nozzle type, a fan pattern of 12 to 45 cm is possible. Thus, nozzles may be selected for each application based on the size and shape of the target surface and the thickness of the coating to be applied. Airless guns create little air turbulence that may repel the liquid from "hard to reach areas", such as would be found in food processing equipment, hatcheries etc. The high flow rate makes airless advantageous in cleaning and disinfecting situations, where the antimicrobial coating is to be applied over a large surface area and multiple surfaces.

The thickness of the applied and dried film will depend on a variety of factors. These factors include the concentration of the film forming agent, the concentration of rheology control additives and/or other additives, as well as the application temperature and humidity. Film thickness and film uniformity also depend, at least in part, on parameters of the application equipment, such as fluid delivery, spray orifice diameter, air pressure or piston pump pressure in the case of airless application, and the distance of the spray applicator to the target surface. Therefore, the liquid formulation may be adjusted to yield the desired film thickness. The atomization of the coating solution is chosen such that a thin film is applied homogeneously to the target area.

Target surfaces (loci) include all surfaces that may potentially be contaminated with microorganisms, including surfaces typically difficult to apply a disinfectant or sanitizer to (such as hard-to-reach surfaces). Examples of target surfaces include equipment surfaces found in the food or beverage industry (such as tanks, conveyors, floors, drains, coolers, freezers, refrigerators, equipment surfaces, ceilings, walls, valves, belts, pipes, drains, ductwork, joints, crevasses, combinations thereof, and the like); building surfaces, including buildings under construction, new home construction, and surfaces in or on seasonal properties like vacation home surfaces (such as ceilings, walls, wood frames, floors, windows, ductwork), kitchens (sinks, drains, counter-tops, refrigerators, cutting boards), bathrooms (showers, toilets, drains, pipes, ductwork, bath-tubs), (especially for mold removal), decks, wood, siding and other home exteriors, asphalt shingle roofing, patio or stone areas (especially for algae treatment); boats and boating equipment surfaces; garbage disposals, garbage cans and dumpsters or other trash removal equipment and surfaces; non-food-industry related pipes and drains; surfaces in hospital, surgery or out-patient centers or veterinary surfaces (such as ceilings, walls, floors, ductwork, beds, equipment, clothing worn in hospital/veterinary or other healthcare settings, including scrubs, shoes, and other hospital or veterinary surfaces) first-responder or other emergency services equipment and clothing; lumber-mill equipment, surfaces and wood products; restaurant surfaces; supermarket, grocery, retail and convenience store equipment and surfaces; deli equipment and surfaces and food preparation surfaces; brewery and bakery surfaces; bathroom surfaces such as sinks, showers, counters, and toilets; clothes and shoes; toys; school and gymnasium equipment, ceilings, walls, floors, windows, ductwork and other surfaces; kitchen surfaces such as sinks, counters, appliances; wooden or composite decks, pool, hot tub and spa surfaces; carpet; paper; leather; animal carcasses, fur and hides; surfaces of barns, or stables for livestock, such as poultry, cattle, dairy cows, goats, horses and pigs; and hatcheries for poultry or for shrimp. Surfaces within structures wherein animals are housed, such as cages and pens for example, can be coated using the antimicrobial coatings described herein. Additional surfaces also include food products, such as beef, poultry, pork, vegetables, fruits, seafood, combinations thereof, and the like.

Additional loci suitable for use in the present invention comprise fibrous substrates and include fibers, yarns, fabrics, textiles, nonwovens, carpets, leather, or paper. The fibrous substrates are made with natural fibers such as wool, cotton, jute, sisal, sea grass, paper, coir and cellulose, or mixtures thereof; or are made with synthetic fibers such as polyamides, polyesters, polyolefins, polyaramids, acrylics and blends thereof; or blends of at least one natural fiber and at least one synthetic fiber. By "fabrics" is meant natural or synthetic fabrics, or blends thereof, composed of fibers such as cotton, rayon, silk, wool, polyester, polypropylene, polyolefins, nylon, and aramids such as "NOMEX®" and "KEVLAR®." By "fabric blends" is meant fabric made of two or more types of fibers. Typically these blends are a combination of at least one natural fiber and at least one synthetic fiber, but also may be a blend of two or more natural fibers or of two or more synthetic fibers. Nonwoven substrates include, for example, spunlaced nonwovens, such as SONTARA available from E. I. du Pont de Nemours and Company (Wilmington, DE, USA), and laminated nonwovens, such as spunbonded-meltblown-spunbonded nonwovens.

Examples of surface materials are metals (e.g., steel, stainless steel, chrome, titanium, iron, copper, brass, aluminum, and alloys thereof), minerals (e.g., concrete), polymers and plastics (e.g., polyolefins, such as polyethylene, polypropylene, polystyrene, poly(meth)acrylate, polyacrylonitrile, polybutadiene, poly(acrylonitrile, butadiene, styrene), poly(acrylonitrile, butadiene), acrylonitrile butadiene; polyesters such as polyethylene terephthalate; and polyamides such as nylon). Additional surfaces include brick, tile, ceramic, porcelain, wood, vinyl, and linoleum.

Equipment or surfaces protected with a temporary coating may be in use or not in use while protected. The target surface may be hydrophobic or hydrophilic.

The coating system may also be one or more components, and may include a catalyst. Generally, the coating is allowed to set or dry for about greater than 5 minutes in order to form the film. However, the coating may be antimicrobially effective in a shorter time-frame, such as after 30 seconds. The coating may be removed before it is dried or anytime thereafter depending on the desired use. The drying time will be partially dependent on a number of factors, including environmental conditions such as humidity and temperature. The drying time will also depend on the thickness of the applied coating.

### Film or coating thickness

The thickness of the film or coating applied onto the target surface influences the time needed for removal and the amount of biocide per unit area applied to the surface. Thicker films increase the time interval until the film has to be re-applied to maintain the desired antimicrobial properties. Thinner films will be easier and faster to remove by rinsing. It is thus important to apply the formulation in a fashion that results in a film thickness that allows both easy removal of the coating and long-lasting antimicrobial properties. As described above, the film or coating has a thickness of about 0.3 to about 300 micrometers. In a more specific embodiment, the film or coating has a thickness of about 0.5 to about 100 micrometers. In an even more specific embodiment, the film or coating has a thickness of about 1.0 to about 30 micrometers.

### Film removal

This method of this invention is directed to films that may be removed at a time determined appropriate by the user. The coatings are removed with ease and may be removed by rinsing the surface with an aqueous solvent or solution. The time of removal may be determined by either (i) the desired minimum contact time to allow for the desired antimicrobial activity, typically expressed as amount of killed or inactivated microorganisms out of a starting population or (ii) the need or desire to take the coating off the surface before starting a subsequent operation or process step. Although the coating may be removed at any time, such as after drying, the film thickness, concentration of antimicrobial agent, and specific use determines the appropriate time for removal. For instance the user may wish to put treated equipment back into normal operation after a period of operational shutdown. Fruits, for example, will require washing prior to eating. Upon exhaustion of the biocide in the film, the film could be removed and a fresh coating layer could be applied. For example, drains may be treated periodically such as daily, weekly or biweekly. Antimicrobial activity may be measured as early as after 30 seconds, hours, days, weeks, months, even years after application of the film. Therefore, timing of removing the coating is a function of the application for which the coating is employed.

Film removal may be achieved by dissolution or dispersion of the resulting coating. This may be achieved by the application of an aqueous solution onto the coating. In one embodiment, the temperature of the solution is in the range of about 15 °C to about 100 °C. In another embodiment, the temperature of the solution is from about 30 to about 80 °C. The application of the solution, or water, may be achieved by a simple rinse or spray onto the surface. Coating removal may also be achieved by use of a pressure washer, facilitating removal by additional mechanical forces. Coating removal may also be achieved by washing with water together with a cloth or sponge. Further, mild additives may be utilized or mixed with the aqueous solution to help solubilize or disperse the film-forming or water-dispersible agents, including commonly used acids or bases, chelators or detergents. Alternatively, the film may be degraded, such as in a drain, by repeated washing of water and/or other components down the drain. The film may also be removed by peeling it off a surface, being abraded or brushed from the surface, or other mechanical mechanisms of removal.

Besides the intentional removal by an operator, removal also includes the removal by an automated or robotic system and the non-intentional removal by a liquid continuously or periodically contacting the coating over time, e.g., in a pipe or drain, or by continuous or periodical application of mechanical forces, such as wear.

### Contact time

Antimicrobial coating compositions of the present invention are effective because they can provide improved contact time with surfaces to be disinfected or sanitized. Contact time refers to the time the coating or coating composition provides antimicrobial properties to microorganisms that come into contact or the vicinity of said coating or coating composition. Depending on the specific requirements for the antimicrobial formulations, the contact time would vary, as set out in Germicidal and Detergent Sanitizing Action of Disinfectants, Official Methods of Analysis of the Association of Official Analytical Chemists, paragraph 960.09 and applicable sections, 15th Edition, 1990 (EPA Guideline 91-2). For example, if the intended application of the present disclosure is use as a sanitizer for food-contact surfaces, then the composition should provide a 99.999% reduction (5-log order reduction) within 30 seconds at room temperature against several test microorganisms. If the intended application is as a sanitizer for non-food contact surfaces, then the composition should provide a 99.9% reduction (3-log order reduction) within 5 minutes at room temperature against several test microorganisms. If the intention is to use the disclosure as a disinfectant, then the composition should provide a 99.9% reduction (3-log order reduction) within 10 minutes. If the intended application is to provide residual antimicrobial activity, then the present method would be allowed to have greater than 10 minute contact time with microorganisms.

### Physical barrier

A physical barrier is defined as the film formed from the present film forming composition. The resulting film seals the treated surface from contamination from the surrounding, such as soil, fat, dust, microorganisms etc. These contaminants will remain on the surface of the coating and will wash off at the time of removal of the coating.

All of the methods and compositions disclosed and claimed herein may be made and executed without undue experimentation in light of the present disclosure. While the methods and compositions of the present disclosure have been described in terms of various aspects of the invention and preferred embodiments, it will be apparent to those of skill in the art that variations may be applied to the compositions and methods and in the steps or in the sequence of steps of the method described herein without departing from the concept, spirit, and scope of the invention. More specifically, it will be apparent that certain agents, which are chemically related, may be substituted for the agents described herein while the same or similar results would be achieved. All such similar substitutes and modifications apparent to those skilled in the art are deemed to be within the spirit, scope, and concept of the invention as defined by the appended claims.

### EXAMPLES

The present invention is further defined in the following Examples. It should be understood that these Examples, while indicating certain preferred embodiments of the invention, are given by way of illustration only. From the above discussion and these Examples, one skilled in the art may ascertain the essential characteristics of this invention, and without departing from the spirit and scope thereof, may make various changes and modifications of the invention to adapt it to various uses and conditions.

### Abbreviations used in the Examples

The following abbreviations are used in the examples: "ATCC" means American Type Culture Collection; "°C" means degrees Celsius; "CFU" means colony forming unit; "FBS" means Fetal bovine serum; "L" means liter; "log CFU" means the base 10 logarithm of the CFU number; "mL" means milliliter; "CFU/mL" means CFU per milliliter; "NFC" means Non-food contact sanitizer test; "Pa*s" means Pascal seconds; "PAA" means peracetic acid; "PEG" means polyethylene glycol; "ppm" means parts per million and refers to mg/L (milligrams per liter) in the following examples; "RPM" means revolutions per minute; "RSS" means residual self-sanitizing activity; "SS316" means stainless steel, type 316 (ASTM standard); "TAED" means tetraacetylethylene diamine; "wt%" means weight percent; "mm" means millimeter; "cm" means centimeter; "g" means gram; "DI water" is deionized water; "min" means minute(s); "µL" means microliter; "%" means percent, "sec" means second(s); "mL/min" means milliliter per minute; "MPa" means megapascal; "g/mol" means gram per mole and "mol%" means mole percent.

### Chemicals

All chemicals were obtained from Sigma-Aldrich (St. Louis, MO, USA) unless stated otherwise. Elvanol® 51-04 (partially hydrolyzed grade polyvinyl alcohol, 88% grade) was from DuPont (Wilmington, DE, USA). Polyethylene glycol (PEG-300) was from Dow (Midland, MI, USA). BTC® 885, Onyxide® 3300 and Biosoft® N25-7 were from Stepan (Northfield, IL, USA). Rheovis® FRC was obtained from Ciba (Basel, Switzerland), TAED B675 was from Warwick International Ltd (Flintshire, U.K.), Provox® C was from OCI Chemical Corp. (Decatur, AL, USA). Glucopon® 215 UP was obtained from Cognis Corporation (Cincinnati, OH). PC 5450 NF was from Performance Chemicals, LLC (Concord, NH). Bacto™ D/E neutralizing broth was from Difco (Cat. No. 281910, Difco™ Laboratories, Detroit, MI, USA).

### GENERAL METHODS

### Test methods for antimicrobial activity on hard surfaces

Peracids may have biocidal activity. Typical alternative biocides known in the art, which may be suitable for use in the present invention include, for example, chlorine, chlorine dioxide, chloroisocyanurates, hypochlorites, ozone, amines, chlorinated phenolics, copper salts, organo-sulphur compounds, and quaternary ammonium salts. Biocidal or antimicrobial efficacy of the coating compositions was measured using the two test methods described below.

*Non-food contact sanitizer test:* To assess the antimicrobial activity of coating compositions for a situation where a microbial contamination was already present on the target surface at the time of the application of the antimicrobial coating composition the "Standard Test Method for Efficacy of Sanitizers Recommended for Inanimate Non-Food Contact Surfaces" according to ASTM standard E1153-03 was used. The test method is referred to as non-food contact sanitizer test or the "NFC test".

*Residual self-sanitizing test*: To assess the antimicrobial activity of coating compositions for a situation where microbial contamination comes into contact with the already dry coating, the following residual self-sanitizing test method was used. The test method is referred to as the residual self-sanitizing test or the "RSS test". Non-porous, pre-cleaned, stainless steel (type SS316) coupons of 25.4 x 25.4 mm in size were used for the test. From a stock plate (transferred three times consecutively but no more than 30 transfers) of the organism, a colony was selected and placed into 10 mL of AOAC Nutrient Broth (NaCl 2.5 g; Beef Extract 2.5 g; Anatone 5 g; Deionized water 500 mL). The inoculated culture was incubated under static conditions for 24 hr at 35°C. In preparation of the test inoculum, the static culture was agitated vigorously using a Vortex mixer, allowed to stand for 15 min, and the upper two-thirds of the culture was transferred to a sterile tube (approximately 6 mL). An organic soil load of fetal bovine serum (FBS) was added for a final concentration of 5 wt% organic soil load to this test inoculum. The final test inoculum density was approximately 1 x 10⁸ CFU/mL.

The test coupons were placed in 70 wt% ethanol overnight, soaked in a mild detergent for at least 30 min, rinsed with tap water thoroughly and allowed to air dry. All handling of surfaces, once cleaned, was done using sterile forceps. Coupons were sprayed with 70 wt% ethanol for 30 min and allowed to dry completely. An aliquot (50 µL) of the coating composition to be tested was applied to each stainless steel coupon, spread evenly with a sterile plastic spreader, placed in a sterile plastic petri dish and allowed to air dry overnight in the biological safety cabinet. Ambient air temperature and relative humidity were recorded. Uncoated coupons were used as control surfaces which were handled under the same conditions as the coupons treated with the coating compositions.

Coupons were inoculated by spotting 0.01 mL of the inoculum using at least 30 spots over the surface of the coated coupon. At least two replicate coupons were inoculated per coating composition. After 5 min, the test coupons were aseptically transferred to 20 mL of Bacto™ D/E neutralizing broth. The tubes were shaken vigorously by hand, sonicated for 10 s in a sonicating water bath at the maximum setting, and finally shaken for 4 min on a rotary shaker at 250 rpm. The cell density of the culture was determined using a serial-dilution spread plate technique with Butterfield Phosphate Buffer dilution tubes and Trypticase™ Soy Agar (TSA) petri plates. All samples were enumerated within approximately 30 min of their transfer to the D/E Neutralizing Broth.

Average cell densities (CFU/mL) for replicate measurements for both treated and untreated coupons were calculated as the geometric mean of the individual CFU measurements. All log numbers are base-10 logarithms.

### Determination of rheological properties

The rheological properties of the liquid antimicrobial formulations was assessed using a Brookfield Digital Viscometer Model DV-II (Brookfield Engineering Laboratories, Middleboro, MA, USA) with RV Series spindle #7 and a tall glass beaker. Samples were loaded by pouring into the glass beaker. Viscosity measurements were taken at different RPM.

### EXAMPLE 1

### TWO-PACK AQUEOUS FILM-FORMING COMPOSITION CONTAINING TWO BIOCIDES

This example illustrates a two-pack aqueous film-forming composition containing two biocides with one biocide being formed *in situ* after components of the two pack system are combined.

The film-forming composition was prepared using the following order of addition with vigorous stirring after each ingredient was added. For preparation of an 80.0 g sample, the following were used:
Pack A: DI water (37.3 g) was added into a 100 mL glass bottle and stirred vigorously. To this was added Rheovis® FRC 1.28 g; Elvanol^{®} 51-04 50.0 g; PEG-300 0.8 g; and the quaternary ammonium compound containing BTC® 885 0.24 g.
Pack B: TAED, B675 0.184 g and sodium percarbonate (Provox C), 0.192 g.

The compositions of Packs A and B in wt% are given in Table 1. Packs A and B after mixing made up 100 wt% of the antimicrobial coating composition. The ratio of Packs A to B was approximately 500:1.

**TABLE 1**

| Coating composition #105 | | |
|---|---|---|
| Pack | Ingredient | wt% |
| A | Elvanol® 51-04 | 10.0 |
| | Rheovis® FRC | 1.6 |
| | PEG-300 | 1.0 |
| | BTC® 885 | 0.3 |
| | Water | 86.6 |
| B | TAED B675 | 0.23 |
| | Sodium percarbonate Provox C | 0.24 |

### EXAMPLE 2

### IN SITU GENERATION OF PERACETIC ACID IN A TWO-PACK FILM-FORMING COMPOSITION WHERE BOTH THE ACTIVATOR AND THE PEROXIDE ARE PRESENT IN THE SAME PACK

This example illustrates the *in situ* generation of peracetic acid in a film-forming composition upon combination of an aqueous Pack A and a solid Pack B from Example 1, where the peracid activator and hydrogen peroxide source are present in Pack B.

Pack A and Pack B from Example 1 were mixed and stirred. The hydrogen peroxide and peracetic acid concentrations were monitored over time using the cerium sulfate/sodium thiosulfate titration test (Greenspan, F. P.; MacKeller, D. G. Anal. Chem., 20: 1061-1063, 1948). Over a one hour time period, the hydrogen peroxide was consumed (from 321 ppm concentration at 5 min to 116 ppm at 60 min), while peracetic acid was generated (increasing from 699 ppm at 5 min to 1073 ppm at 60 min). Table 2 shows the concentrations of hydrogen peroxide and PAA over time.

**TABLE 2**

| H₂O₂ and PAA concentrations over time for coating composition #105 | | | | |
|---|---|---|---|---|
| | **5 min** | **10 min** | **30 min** | **60 min** |
| H₂O₂ ppm | 321 | 247 | 159 | 116 |
| PAA ppm | 699 | 822 | 1031 | 1073 |

### EXAMPLE 3

### IN SITU GENERATION OF PERACETIC ACID IN A TWO-PACK FILM-FORMING COMPOSITION WHERE THE ACTIVATOR AND THE PEROXIDE ARE SEPARATED IN TWO PACKS

This example illustrates the *in situ* generation of PAA in a film-forming composition upon combination of an aqueous Pack A and a solid Pack B, where the peracid activator is in Pack A and the hydrogen peroxide source is present in Pack B.

Pack A and Pack B (composition #85, see Table 3) were mixed and stirred. The hydrogen peroxide and peracetic acid concentrations were monitored over time using the cerium sulfate/sodium thiosulfate titration method described above. Over a two hour time period, the hydrogen peroxide was consumed, while production of peracetic acid was observed. The compositions of Pack A and B in wt% are given in Table 3. The wt% numbers in the table are defined as the mass of the component divided by the mass of the combined Pack A and B. The ratio of Pack A to B was approximately 500:1. Table 4 shows the concentrations of hydrogen peroxide and PAA over time underlining decrease in the hydrogen peroxide level from 230 ppm at 5 min to 34 ppm in 120 min while PAA increased from 734 ppm at 5 min to 982 ppm in 120 min.

**TABLE 3**

| Coating composition of #85 | | |
|---|---|---|
| Pack | Ingredient | wt% |
| A | Elvanol® 51-04 | 10.0 |
| | Rheovis® FRC | 1.6 |
| | PEG-300 | 1.0 |
| | BTC® 885 | 0.3 |
| | TAED | 0.23 |
| | Water | 86.7 |
| B | Sodium percarbonate | 0.21 |

**TABLE 4**

| H₂O₂ and PAA concentrations over time for coating composition #85 | | | |
|---|---|---|---|
| | 5 min | 30 min | 120 min |
| H₂O₂ ppm | 230 | 85 | 34 |
| PAA ppm | 735 | 1080 | 982 |

### EXAMPLE 4

### Add dry film example: in situ versus commercial PAA

### EXAMPLE 5

### THE EFFICACY OF THE FILM-FORMING ANTIMICROBIAL COMPOSITION #92 ON KLEBSIELLA PNEUMONIAE

The antimicrobial properties of the film-forming composition #92 (Table 5) were tested using the RSS test method and *Klebsiella pneumoniae* (ATCC 4352) as the test microorganism. The composition of #92 in wt% is given in Table 5. The wt% numbers in the table are defined as the mass of the component divided by the mass of the combined Pack A and B. The mass ratio of Pack A to B is approximately 500:1. A volume of 0.05 mL of the coating composition was applied evenly per coupon. As shown in Table 5, a 3.6 log reduction in CFU was achieved for sample #92, which is equivalent to a reduction of the CFU number by more than 99.97%.

**TABLE 5**

| Antimicrobial RSS test with *K. pneumoniae* | | |
|---|---|---|
| Pack | Ingredient (wt%) | #92 |
| A | Elvanol® 51-04 | 10 |
| | Rheovis® FRC | 1.6 |
| | PEG-300 | 5.2 |
| | TAED | 0.23 |
| | Onyxide® 3300 | 0.4 |
| | Water | 82.1 |
| B | Sodium percarbonate | 0.21 |
| | Contact time (min) | 5 |
| | Log₁₀ CFU reduction | 3.6 |

### EXAMPLE 6

### THE EFFICACY OF THE FILM-FORMING ANTIMICROBIAL COMPOSITION #92 ON STAPHYLOCOCCUS AUREUS

The antimicrobial properties of the film-forming composition #92 (Table 6) were tested using the NFC test method and *Staphylococcus aureus* (ATCC 6538 as the test microorganism. The composition of #92 in wt% is given in Table 6. Combined Pack A and B after mixing were 100 wt%. The mass ratio of Pack A to B was approximately 500:1. A volume of 0.05 mL of the coating composition was applied evenly per coupon. As shown in Table 6, a 4.5 log reduction in colony-forming units (CFU) or greater was, which is equivalent to a reduction of the CFU number by at least 99.99%.

**TABLE 6**

| Antimicrobial NFC Test with *S*. *aureus* | | |
|---|---|---|
| Pack | Ingredient (wt%) | #92 |
| A | Elvanol® 51-04 | 10 |
| | Rheovis® FRC | 1.6 |
| | PEG-300 | 5.2 |
| | TAED | 0.23 |
| | Onyxide® 3300 | 0.4 |
| | Water | 82.1 |
| B | Sodium percarbonate | 0.21 |
| | Contact time (min) | 5 |
| | Log₁₀ CFU reduction | 4.5 |

### EXAMPLE 7

### THE EFFICACY OF THE FILM-FORMING ANTIMICROBIAL COMPOSITION #105 ON STAPHYLOCOCCUS AUREUS

The antimicrobial properties of the film-forming composition #105 (Table 1) were tested using the NFC test method and *Staphylococcus aureus* (ATCC 6538) as the test microorganism. The composition of #105 in wt% is given in Table 1. Combined Pack A and B after mixing were 100 wt%. The mass ratio of Pack A to B was approximately 500:1. A volume of 0.05 mL of the coating composition was applied evenly per coupon. With a five minute exposure, a 5.6 log reduction in colony-forming units (CFU) or greater was observed, which is equivalent to a reduction of the CFU number by at least 99.999%.

### EXAMPLE 8

### THE EFFICACY OF THE FILM-FORMING ANTIMICROBIAL COMPOSITION #139 ON STAPHYLOCOCCUS AUREUS

The antimicrobial properties of the film-forming composition #139 (Table 7) were tested using the RSS test method and *Staphylococcus aureus* (ATCC 6538) as the test microorganism. The composition of #139 in wt% is given in Table 7. Combined Pack A and B after mixing were 100 wt%. The mass ratio of Pack A to B is approximately 500:1. A volume of 0.05 mL of the coating composition was applied evenly per coupon. As shown in Table 7, a 3.0 log reduction in CFU was achieved for sample #139, which is equivalent to a reduction of the CFU number by at least 99.9%.

**TABLE 7**

| Antimicrobial RSS test with *S*. *aureus* | | |
|---|---|---|
| Pack | Ingredient (wt%) | #139 |
| A | Elvanol® 51-04 | 10 |
| | Rheovis® FRC | 2.2 |
| | PEG-300 | 1.0 |
| | Glucopon® 215 UP | 0.5 |
| | BTC® 885 | 0.3 |
| | PC 5450 NF | 0.2 |
| | Biosoft® N25-7 | 0.01 |
| | Water | 84.4 |
| B | TAED B675 | 0.69 |
| | Provox® C | 0.72 |
| | Contact time (min) | 5 |
| | Log₁₀ CFU reduction | 3.0 |

### EXAMPLE 9

The "pseudoplastic index" or "shear thinning index" (STI) provides an indication as to the composition's resistance to sagging and dripping. A common measurement determines the viscosity at two different shear rates such as 1 s⁻¹ and 10 s⁻¹. The value recorded at the lower shear rate is divided by the value at the higher shear rate obtain the STI. Generally, the higher the STI, the higher the resistance to sagging and dripping the coating material will have.

The STI values as used in herein were determined by measuring the viscosities after mixing the two packs at two different shear-rates of 1 s⁻¹ and 10 s⁻¹. The viscosities were measured using a Bohlin Gemini controlled-stress rheometer (Malvern Instruments Ltd., Worcestershire, UK). The instrument was equipped with a peltier heating system and a 40 mm parallel plate with smooth surfaces. The distance between the plates, called the "gap" was adjusted to 0.150 mm. The peltier was set at the desired test temperature of 10 °C. Less than 1 mL of sample was added to the peltier plate. The upper parallel plate was lowered to the desired gap. The excess material was first removed with a pipette and then the straight edge of a piece of plastic was used to cleanly trim the sample around the parallel plate. The sample was pre-sheared for 30 s at a shear rate of 2000 s⁻¹ and then allowed to recover while the instrument reached the temperature set point. A shear rate sweep was performed from 0.03-30,000 s⁻¹ over the course of 400 s. The shear-thinning index (STI) was calculated by dividing the viscosity measured at 1 s⁻¹ by the viscosity measured at 10 s⁻¹. The STI values for two formulations according to this invention (#115D and E; see Table 7 for composition) are given in Table 8.

**TABLE 8**

| Coating composition of #115D and E | | | |
|---|---|---|---|
| Pack | Ingredient | #115D wt% | #115D wt% |
| A | Elvanol® 51-04 | 10 | 10 |
| | Rheovis® FRC | 2.2 | 2.4 |
| | PEG-300 | 1.0 | 1.0 |
| | BTC® 885 | 0.3 | 0.3 |
| | Water | 86.0 | 85.8 |
| B | TAED B675 | 0.23 | 0.23 |
| | Sodium percarbonate Provox C | 0.24 | 0.24 |

**TABLE 9**

| Viscosities and shear-thinning index for #115D and #115E | | | | |
|---|---|---|---|---|
| Sample | T (°C) | Viscosity at1 s⁻¹ (Pa*s) | Viscosity at 10 s⁻¹ (Pa*s) | STI |
| #115D | 10 | 10.94 | 3.53 | 3.10 |
| #115E | 10 | 12.5 | 3.86 | 3.24 |

### EXAMPLE 10

### SPRAY APPLICATION OF COATING COMPOSITION #248 USING AIRLESS SPRAY EQUIPMENT

Coating composition #248 was applied to surfaces by spraying using an airless spray system (model President 46/1, Graco Inc., Minneapolis, MN, USA). A liquid pressure of 31.7 MPa was used which resulted in excellent sprayability characteristics such efficient atomization, complete coverage and low tendency to sag or drip off vertical surfaces. The sag point is defined as the thickness of the coating after spraying on a vertical surface and drying at which the coatings starts to show visual sags or drips. The sag point was measured to be above 10 micrometers for coating composition #248 indicating a high resistance to sagging and dripping. The resulting coating after drying had an excellent appearance characterized by the absence of coating defects such as sags, foam or bubbles, craters or uncovered areas.

The application speed of the coating composition was measured to be about 8 to 15 m²/min depending on the speed of moving the spray gun across the surface to be sprayed. The consumption of the coating composition was between about 30 and 60 g/m², again depending on the speed of moving the spray gun across the target surface.

## Claims

1. A method of providing control of microorganisms at a locus comprising the steps:
a) forming a composition by combining components comprising:
i) a water soluble or water-dispersible film-forming agent selected from polyvinyl alcohol, polyvinyl alcohol copolymer or polyvinyl pyrrolidone
ii) an inert solvent;
iii) a peroxyacid precursor;
iv) a peroxygen source;
v) a rheology modifier to provide shear-thinning properties; and
vi) an antimicrobial agent comprising a quaternary ammonium compound
to form an antimicrobial liquid coating composition comprising at least one peroxyacid antimicrobial agent; and
b) applying said composition obtained in step (a) to said locus; and
c) allowing said composition to dry thereby forming a coating on said locus,
wherein the shear-thinning index of the liquid coating composition is between 1.5 and 6.0.

2. The method according to Claim 1, wherein the viscosity of the liquid coating composition is between 0.5 and 100 Pa*s, as measured at 10 °C and a shear rate of 1 s⁻¹.

3. The method of Claim 1, further comprising d) removing said coating.

4. The method of Claim 1, wherein at least one or more said components are separately packaged from the other components in a multi-compartment system prior to their combination to form said liquid coating composition.

5. A method of providing control of microorganisms at a locus comprising the steps:
a) combining the contents of a first premixed component comprising an inert solvent and a film-forming agent selected from polyvinyl alcohol, polyvinyl alcohol copolymer or polyvinyl pyrrolidone, with the contents of a second premixed component comprising a peroxyacid precursor and a peroxygen source to form an antimicrobial liquid coating composition comprising at least one peroxyacid antimicrobial agent;
b) applying said composition obtained in step (a) to the locus; and
c) allowing said coating composition to dry thereby forming a coating on said locus;
wherein at least one premixed component further comprises a second antimicrobial agent wherein said second antimicrobial agent comprises a quaternary ammonium compound, and wherein at least one premixed component further comprises at least one rheology agent that provides shear-thinning properties to the coating composition and
wherein the shear-thinning index of the liquid coating composition is between 1.5 and 6.0.

6. The method according to Claim 5, wherein the viscosity of the liquid coating composition is between 0.5 and 100 Pa*s, as measured at 10 °C and a shear rate of 1 s⁻¹.

7. The method of Claim 5, wherein said first and second premixed components are provided in a multi-compartment system; wherein the first and second premixed components remain separated before formation of the liquid coating composition.

8. An antimicrobial composition comprising components which comprise:
a) a water soluble or water-dispersible film-forming agent selected from polyvinyl alcohol, polyvinyl alcohol copolymer or polyvinyl pyrrolidone
b) an inert solvent;
c) a peroxyacid precursor;
d) a rheology modifier to provide shear-thinning properties;
e) an antimicrobial agent comprising a quarternary ammonium compound; and
f) a peroxygen source;
wherein, upon combining said components, a peroxyacid is formed; wherein the shear-thinning index of the antimicrobial composition is between 1.5 and 6.0
optionally wherein at least one or more said components are separately packaged from the other components in a multi-compartment system prior to their combination to form said peroxyacid.

9. The composition of Claim 8, wherein said composition provides a reduction of colony-forming units of at least 99.9% when applied to a contaminated surface.

10. The composition of Claim 8, wherein the peroxyacid precursor is selected from the group consisting of N-acyl amides or O-acyl esters; and the peroxygen source is hydrogen peroxide.

11. An article comprising a coating on at least one surface thereof of a removable antimicrobial composition, wherein the antimicrobial composition comprises:
a) a water soluble or water-dispersible film-forming agentselected from polyvinyl alcohol, polyvinyl alcohol copolymer or polyvinyl pyrrolidone
b) an inert solvent;
c) a peroxyacid precursor;
d) a peroxygen source;
e) an antimicrobial agent comprising a quarternary ammonium compound; and
f) a rheology modifier to provide shear-thinning properties,
wherein the shear-tinning index of the antimicrobial composition is between 1.5 and 6.0 and
wherein, upon combining components of (a) through (d), a peroxyacid is formed.

12. The article of Claim 11 wherein the article is equipment used in the food or beverage industry.

## Patentansprüche

1. Verfahren zum Bereitstellen der Bekämpfung von Mikroorganismen an einer Stelle, umfassend die Schritte des:
a) Bildens einer Zusammensetzung durch Kombinieren von Komponenten umfassend:
i) ein wasserlösliches oder wasserdispergierbares, filmbildendes Mittel ausgewählt unter Polyvinylalkohol, Polyvinylalkoholcopolymer oder Polyvinylpyrrolidon;
ii) ein inertes Lösungsmittel;
iii) einen Peroxysäurevorläufer;
iv) eine Persauerstoffquelle;
v) ein Rheologiemodifiziermittel, um strukturviskose Eigenschaften zu verleihen; und
vi) ein antimikrobielles Mittel umfassend eine quartäre Ammoniumverbindung,
um eine antimikrobielle flüssige Beschichtungszusammensetzung zu bilden, die mindestens ein antimikrobielles Peroxysäuremittel umfasst; und
b) Aufbringens der in Schritt (a) erhaltenen Zusammensetzung auf eine Stelle; und
c) Gestattens, dass die Zusammensetzung trocken wird und dabei eine Beschichtung auf der Stelle bildet,
wobei der Strukturviskositätsindex der flüssigen Beschichtungszusammensetzung 1,5 bis 6,0 beträgt.

2. Verfahren nach Anspruch 1, wobei die Viskosität der flüssigen Beschichtungszusammensetzung 0,5 bis 100 Pa·s, wie bei 10 °C und einer Scherrate von 1 s⁻¹ gemessen, beträgt.

3. Verfahren nach Anspruch 1, des Weiteren d) das Entfernen der Beschichtung umfassend.

4. Verfahren nach Anspruch 1, wobei mindestens eine oder mehrere der Komponenten von den anderen Komponenten getrennt in einem Multikompartimentsystem vor Kombinieren derselben zur Bildung der flüssigen Beschichtungszusammensetzung verpackt werden.

5. Verfahren zum Bereitstellen der Bekämpfung von Mikroorganismen an einer Stelle, umfassend die Schritte des:
a) Kombinierens der Inhalte einer ersten vorgemischten Komponente umfassend ein inertes Lösungsmittel und ein filmbildendes Mittel ausgewählt unter Polyvinylalkohol, Polyvinylalkoholcopolymer oder Polyvinylpyrrolidon mit den Inhalten einer zweiten vorgemischten Komponente umfassend einen Peroxysäurevorläufer und eine Persauerstoffquelle unter Bildung einer antimikrobiellen flüssigen Beschichtungszusammensetzung umfassend mindestens ein antimikrobielles Peroxysäuremittel; und
b) Aufbringens der in Schritt (a) erhaltenen Zusammensetzung auf die Stelle; und
c) Gestattens, dass die Beschichtungszusammensetzung trocken wird und dabei eine Beschichtung auf der Stelle bildet,
wobei mindestens eine vorgemischte Komponente des Weiteren ein zweites antimikrobielles Mittel umfasst, wobei das zweite antimikrobielle Mittel eine quartäre Ammoniumverbindung umfasst und wobei mindestens eine vorgemischte Komponente des Weiteren mindestens ein Rheologiemittel umfasst, das der Beschichtungszusammensetzung strukturviskose Eigenschaften verleiht und wobei der Strukturviskositätsindex der flüssigen Beschichtungszusammensetzung 1,5 bis 6,0 beträgt.

6. Verfahren nach Anspruch 5, wobei die Viskosität der flüssigen Beschichtungszusammensetzung 0,5 bis 100 Pa·s, wie bei 10 °C und einer Scherrate von 1 s⁻¹ gemessen, beträgt.

7. Verfahren nach Anspruch 5, wobei die ersten und zweiten vorgemischten Komponenten in einem Multikompartimentsystem bereitgestellt werden; wobei die ersten und zweiten vorgemischten Komponenten vor Bildung der flüssigen Beschichtungszusammensetzung getrennt bleiben.

8. Antimikrobielle Zusammensetzung umfassend Komponenten, die Folgendes umfassen:
a) ein wasserlösliches oder wasserdispergierbares, filmbildendes Mittel ausgewählt unter Polyvinylalkohol, Polyvinylalkoholcopolymer oder Polyvinylpyrrolidon;
b) ein inertes Lösungsmittel;
c) einen Peroxysäurevorläufer;
d) ein Rheologiemodifiziermittel, um strukturviskose Eigenschaften zu verleihen;
e) ein antimikrobielles Mittel umfassend eine quartäre Ammoniumverbindung; und
f) eine Persauerstoffquelle;
wobei auf das Kombinieren der Komponenten hin eine Peroxysäure gebildet wird;
wobei der Strukturviskositätsindex der antimikrobiellen Beschichtungszusammenetzung 1,5 bis 6,0 beträgt,
wobei wahlweise mindestens eine oder mehrere der Komponenten von den anderen Komponenten getrennt in einem Multikompartimentsystem vor Kombinieren derselben zur Bildung der Peroxysäure verpackt werden.

9. Zusammensetzung nach Anspruch 8, wobei die Zusammensetzung eine Reduktion koloniebildender Einheiten von mindestens 99,9 %, wenn auf eine kontaminierte Oberfläche aufgebracht, bereitstellt.

10. Zusammensetzung nach Anspruch 8, wobei der Peroxysäurevorläufer aus der Gruppe ausgewählt ist bestehend aus N-Acylamiden oder O-Acylestern; und die Persauerstoffquelle Wasserstoffperoxid ist.

11. Artikel umfassend eine Beschichtung auf mindestens einer Oberfläche davon aus einer entfernbaren antimikrobiellen Zusammensetzung, wobei die antimikrobielle Zusammensetzung Folgendes umfasst:
a) ein wasserlösliches oder wasserdispergierbares, filmbildendes Mittel ausgewählt unter Polyvinylalkohol, Polyvinylalkoholcopolymer oder Polyvinylpyrrolidon
b) ein inertes Lösungsmittel;
c) einen Peroxysäurevorläufer;
d) eine Persauerstoffquelle;
e) ein antimikrobielles Mittel umfassend eine quartäre Ammoniumverbindung; und
f) ein Rheologiemodifiziermittel, um strukturviskose Eigenschaften zu verleihen,
wobei der Strukturviskositätsindex der antimikrobiellen Zusammensetzung 1,5 bis 6,0 beträgt und
wobei auf das Kombinieren der Komponenten von (a) bis (d) hin eine Peroxysäure gebildet wird.

12. Artikel nach Anspruch 11, wobei der Artikel eine Vorrichtung ist, die in der Nahrungsmittel- oder Getränkeindustrie verwendet wird.

## Revendications

1. Procédé de fourniture du contrôle de micro-organismes au niveau d'un lieu comprenant les étapes:
a) de formation d'une composition en combinant des composants comprenant:
i) un agent formant film soluble dans l'eau ou pouvant être dispersé dans l'eau sélectionné parmi le poly(alcool de vinyle), le copolymère de poly(alcool de vinyle) ou la polyvinyl pyrrolidone;
ii) un solvant inerte;
iii) un précurseur de peroxyacide;
iv) une source de peroxygène;
v) un agent de modification de la rhéologie pour fournir des propriétés de rhéo-fluidification; et
vi) un agent antimicrobien comprenant un composé d'ammonium quaternaire;
pour former une composition de revêtement liquide antimicrobienne comprenant au moins un agent antimicrobien de type peroxyacide; et
b) d'application de ladite composition obtenue dans étape (a) audit lieu; et
c) permettant de laisser ladite composition sécher formant de là un revêtement sur ledit lieu,
dans lequel l'indice de rhéo-fluidification de la composition de revêtement liquide est compris entre 1,5 et 6,0.

2. Procédé selon la revendication 1, dans lequel la viscosité de la composition de revêtement liquide est comprise entre 0,5 et 100 Pa*s, telle que mesurée à 10°C et une vitesse de cisaillement de 1 s⁻¹.

3. Procédé selon la revendication 1, comprenant en outre d) l'élimination dudit revêtement.

4. Procédé selon la revendication 1, dans lequel au moins un ou plusieurs desdits composants est(sont) conditionné(s) séparément des autres composants dans un système à compartiments multiples avant leur combinaison pour former ladite composition de revêtement liquide.

5. Procédé de fourniture du contrôle de micro-organismes au niveau d'un lieu comprenant les étapes:
a) de combinaison des contenus d'un premier composant prémélangé comprenant un solvant inerte et un agent formant film sélectionné parmi le poly(alcool de vinyle), le copolymère de poly(alcool de vinyle) ou la polyvinyl pyrrolidone, avec les contenus d'un second composant prémélangé comprenant un précurseur de peroxyacide et une source de peroxygène pour former une composition de revêtement liquide antimicrobienne comprenant au moins un agent antimicrobien de type peroxyacide;
b) d'application de ladite composition obtenue dans l'étape (a) au niveau du lieu; et
c) permettant de laisser ladite composition de revêtement sécher formant de là un revêtement sur ledit lieu;
dans lequel au moins un composant prémélangé comprend en outre un second agent antimicrobien dans lequel ledit second agent antimicrobien comprend un composé d'ammonium quaternaire, et dans lequel au moins un composant prémélangé comprend en outre au moins un agent de rhéologie qui fournit des propriétés de rhéo-fluidification à la composition de revêtement et dans lequel l'indice de rhéo-fluidification de la composition de revêtement liquide est compris entre 1,5 et 6,0.

6. Procédé selon la revendication 5, dans lequel la viscosité de la composition de revêtement liquide est comprise entre 0,5 et 100 Pa*s, telle que mesurée à 10°C et une vitesse de cisaillement de 1 s⁻¹.

7. Procédé selon la revendication 5, dans lequel lesdits premier et second composants prémélangés sont présentés dans un système à compartiments multiples; dans lequel le premier et le second composants prémélangés restent séparés avant la formation de la composition de revêtement liquide.

8. Composition antimicrobienne comprenant des composants qui comprennent:
a) un agent formant film soluble dans l'eau ou pouvant être dispersé dans l'eau sélectionné parmi le poly(alcool de vinyle), le copolymère de poly(alcool de vinyle) ou la polyvinyl pyrrolidone;
b) un solvant inerte;
c) un précurseur de peroxyacide;
d) un agent de modification de la rhéologie pour fournir des propriétés de rhéo-fluidification;
e) un agent antimicrobien comprenant un composé d'ammonium quaternaire; et
f) une source de peroxygène;
dans laquelle, à l'issue de la combinaison desdits composants, un peroxyacide est formé; dans laquelle l'indice de rhéo-fluidification de la composition antimicrobienne est compris entre 1,5 et 6,0;
optionnellement dans laquelle au moins un ou plusieurs desdits composants est(sont) conditionné(s) séparément des autres composants dans un système à compartiments multiples avant leur combinaison pour former ledit peroxyacide.

9. Composition selon la revendication 8, dans laquelle ladite composition fournit une réduction des unités formant colonies d'au moins 99,9 % lorsqu'appliquée à une surface contaminée.

10. Composition selon la revendication 8, dans laquelle le précurseur de peroxyacide est sélectionné parmi le groupe constitué des N-acyl amides ou des esters de O-acyle; et la source de peroxygène est le peroxyde d'hydrogène.

11. Article comprenant un revêtement sur au moins une surface de celui-ci d'une composition antimicrobienne pouvant être retirée, dans lequel la composition antimicrobienne comprend:
a) un agent formant film soluble dans l'eau ou pouvant être dispersé dans l'eau sélectionné parmi le poly(alcool de vinyle), le copolymère de poly(alcool de vinyle) ou la polyvinyl pyrrolidone;
b) un solvant inerte;
c) un précurseur de peroxyacide;
d) une source de peroxygène;
e) un agent antimicrobien comprenant un composé d'ammonium quaternaire; et
f) un agent de modification de la rhéologie pour fournir des propriétés de rhéo-fluidification,
dans lequel l'indice de rhéo-fluidification de la composition antimicrobienne est compris entre 1,5 et 6,0 et
dans lequel, à l'issue de la combinaison des composants (a) jusqu'à (d), un peroxyacide est formé.

12. Article selon la revendication 11 dans lequel l'article est un équipement utilisé dans l'industrie alimentaire ou des boissons.
